# EUROPEAN PATENT APPLICATION

(11) **EP 4 130 239 A1**
(43) Date of publication of application: **08.02.2023**
(21) Application number: 21776837.3
(22) Date of filing: 18.03.2021
(51) Int. Cl.: C12N 5/071, C12N 5/0775

(54) **METHOD FOR INDUCING DIFFERENTIATION INTO PANCREATIC ALPHA CELLS**

(30) Priority: 24.03.2020 JP 2020052420
(71) Applicant: Kaneka Corporation, Osaka-shi, Osaka 530-8288 (JP); National Center for Global Health and Medicine, Tokyo 162-8655 (JP)
(72) Inventor: KIMURA Keiichi, Kobe-shi, Hyogo 650-0047 (JP); IBUKI Masato, Kobe-shi, Hyogo 650-0047 (JP); OKOCHI Hitoshi, Tokyo 162-8655 (JP); YABE Shigeharu, Tokyo 162-8655 (JP)
(74) Representative: Hoffmann Eitle
(86) International application number: PCT/JP2021/011125
(87) International publication number: WO 2021/193360

(57) **Abstract**

A method for inducing differentiation into pancreatic α cells includes: a step (a) of culturing endodermal cells, which have been induced to differentiate from pluripotent stem cells, in the presence of a bone morphogenetic protein (BMP) signaling inhibitor, and retinoic acid or a retinoic acid analog to induce differentiation into primitive gut tube (PGT) cells; a step (b) of culturing the primitive gut tube (PGT) cells to induce differentiation into pancreatic endocrine precursor (EP) cells; and a step (c) of culturing the pancreatic endocrine precursor (EP) cells to induce differentiation into pancreatic α cells, in which the step (b) and the step (c) are performed in the absence of ascorbic acid.

## Description

### TECHNICAL FIELD

The present invention relates to a method for inducing differentiation into pancreatic α cells.

Priority is claimed on Japanese Patent Application No. 2020-52420, filed March 24, 2020, the content of which is incorporated herein by reference.

### BACKGROUND ART

Considerable expectations are being placed on regenerative medicine as an alternative to organ transplantation, which has a donor shortage issue, and in the development of new therapies for intractable diseases and the like. Embryonic stem cells (ES cells) and induced pluripotent stem cells (iPS cells) have pluripotency and infinite proliferative capacity, and are thus expected to be able to serve as cell sources for preparing the cells required for regenerative medicine. In order to put regenerative medicine using such pluripotent stem cells into practice, a technique for efficiently inducing the differentiation of pluripotent stem cells into target somatic cells needs to be established, and various differentiation induction methods have been reported.

Pancreatic islets (islets of Langerhans) are endocrine tissues present inside the pancreas, secrete insulin and glucagon, and mainly regulate blood glucose. The pancreatic islets are composed of various cells, and α cells, β cells, δ cells, and the like are present. Among them, pancreatic β cells are useful in cell therapy for diabetes. Thus, methods for efficiently producing pancreatic β cells from pluripotent stem cells have been explored. Patent Document 1 discloses a method for producing pancreatic β cells from endodermal cells induced to differentiate from pluripotent stem cells.

In recent years, it has been reported that, in the pancreatic islets of diabetic patients, abnormalities occur not only in pancreatic β cells but also in pancreatic α cells that promote gluconeogenesis in response to low blood glucose. In such patients, pancreatic α cells are in an active state even in a high blood glucose condition, and continue to secrete glucagon, which raises a blood glucose level. Thus, it has been known that glucagon secreted from pancreatic α cells plays an important role in the regulation of blood glucose levels in a living body, and there are great expectations for elucidation of the glucagon-secretory mechanism from the viewpoint of developing a novel therapeutic agent for diabetes.

However, there is a chronic shortage of pancreatic α cells provided for research, and it is necessary to secure a new source for pancreatic α cells in order to accelerate this research. Since pluripotent stem cells such as iPS cells and ES cells can differentiate into almost all cells constituting a living body, they are expected to be new cell sources for pancreatic α cells. In order to put pancreatic α cells derived from pluripotent stem cells into practical use, a technique for efficiently inducing differentiation of pluripotent stem cells into functional pancreatic α cells is required. Non-Patent Document 1 reports a method for inducing differentiation of human embryonic stem cells into pancreatic α cells having a glucagon-secretory function. However, a technique for efficiently inducing differentiation of pluripotent stem cells into functional pancreatic α cells has not been reported much so far, and a fully functional pancreatic α cell line has not been established.

### Citation List

### Patent Document

[Patent Document 1]
PCT International Publication No. WO2019/208788

### Non-Patent Document

[Non-Patent Document 1]
Alireza Rezania et al., Diabetes, 2011 Jan; 60(1): 239-247

### DISCLOSURE OF INVENTION

### Technical Problem

As mentioned above, from the viewpoint of elucidating the mechanism of glucagon secretion from pancreatic α cells, constructing a development tool of a new therapeutic agent for diabetes, and the like, it is necessary to provide a technique for efficiently inducing the differentiation of pluripotent stem cells into functional pancreatic α cells.

Accordingly, an object of the present invention is to provide a method for efficiently inducing differentiation of pluripotent stem cells into functional pancreatic α cells.

### Solution to Problem

As a result of diligent investigations towards achieving the above-mentioned object, the present inventors discovered that, by culturing endodermal cells, which had been induced to differentiate from pluripotent stem cells, under prescribed conditions, the endodermal cells can be induced to differentiate into pancreatic α cells, and the obtained pancreatic α cells have a glucagon-secretory function. The present invention was completed on the basis of this discovery.

In other words, according to the present description, the following invention is provided.
[1] A method for inducing differentiation into pancreatic α cells, the method including:
   a step (a) of culturing endodermal cells, which have been induced to differentiate from pluripotent stem cells, in the presence of a bone morphogenetic protein (BMP) signaling inhibitor, and retinoic acid or a retinoic acid analog to induce differentiation into primitive gut tube (PGT) cells;
   a step (b) of culturing the primitive gut tube (PGT) cells to induce differentiation into pancreatic endocrine precursor (EP) cells; and
   a step (c) of culturing the pancreatic endocrine precursor (EP) cells to induce differentiation into pancreatic α cells,
   in which the step (b) and the step (c) are performed in the absence of ascorbic acid.
[2] The method for inducing differentiation into pancreatic α cells according to [1], in which the step (c) is performed under an oxygen supply condition.
[3] The method for inducing differentiation into pancreatic α cells according to [2], in which the oxygen supply condition is a condition in which a dissolved oxygen concentration in a culture solution is controlled to be 20% to 50% when a saturated dissolved oxygen concentration in the culture solution at 37°C at 1 atm is 100%.
[4] The method for inducing differentiation into pancreatic α cells according to any one of [1] to [3], in which the step (a) is performed in the presence of a ROCK signaling inhibitor.
[5] The method for inducing differentiation into pancreatic α cells according to any one of [1] to [4], in which the endodermal cells induced to differentiate from the pluripotent stem cells are endodermal cells induced to differentiate by culturing the pluripotent stem cells in a culture medium to which FGF2 and BMP4 are not added after culturing in a culture medium containing a TGFβ superfamily signaling activator.
[6] The method for inducing differentiation into pancreatic α cells according to any one of [1] to [5],
   in which the step (b) includes
   a step (b1) of culturing the primitive gut tube (PGT) cells in the presence of a protein kinase C (PKC) activator to induce differentiation into posterior foregut (PFG) cells,
   a step (b2) of culturing the posterior foregut (PFG) cells in the presence of retinoic acid or an analog thereof to induce differentiation into pancreatic progenitor (PP) cells, and
   a step (b3) of culturing the pancreatic progenitor (PP) cells in the presence of a Notch signaling inhibitor and a ROCK signaling inhibitor to induce differentiation into pancreatic endocrine precursor (EP) cells.
[7] The method for inducing differentiation into pancreatic α cells according to any one of [1] to [6], in which the step (c) includes a step of culturing the pancreatic endocrine precursor (EP) cells in the presence of an insulin receptor signaling activator, transferrin, and selenous acid to induce differentiation into pancreatic α cells.
[8] The method for inducing differentiation into pancreatic α cells according to any one of [1] to [7], in which the culturing is performed by suspension culture.
[9] The method for inducing differentiation into pancreatic α cells according to any one of [2] to [8], in which the oxygen supply is performed under stirring culture.

### Advantageous Effects of Invention

By the method for inducing differentiation into pancreatic α cells according to present invention, pluripotent stem cells can be efficiently induced to differentiate into functional pancreatic α cells. In addition, the pancreatic α cells produced by the present invention are useful for elucidating the glucagon-secretory mechanism and as a development tool of a novel therapeutic agent for diabetes.

### BRIEF DESCRIPTION OF DRAWINGS

Fig. 1 shows the results of analyzing the expression of an INS gene, a GCG gene, an NKX6.1 gene, and a PDX1 gene in cells obtained by methods of Example 1 and Comparative Example 1.
Fig. 2 shows the measurement results of the glucagon secretion amount of cells obtained by inducing differentiation of primitive gut tube (PGT) cells, which had been induced to differentiate from human iPS cells, in the presence or absence of ascorbic acid.
Fig. 3 shows the measurement results of the glucagon secretion amount of cells obtained by a method of Example 2 and the cells obtained by the method of Comparative Example 1.
Fig. 4 shows the results of analyzing the expression of a GCG gene, an ARX gene, a GC gene, an INS gene, and an NKX6.1 gene in the cells obtained by the method of Example 2 and the cells obtained by the method of Comparative Example 1.
Fig. 5 shows the oxygen concentration in a culture solution when culturing under the controlled oxygen concentration of #3-1 in a step of culturing pancreatic endocrine precursor (EP) cells to induce differentiation into pancreatic α cells.
Fig. 6 shows the results of analyzing the expression of a GCG gene in cells obtained by culturing pancreatic endocrine precursor (EP) cells under the controlled oxygen concentration (#3-1 to #3-3) or under the uncontrolled oxygen concentration (Comparative Example 1) to induce differentiation.
Fig. 7 shows the results of analyzing the expression of an INS gene in cells obtained by culturing pancreatic endocrine precursor (EP) cells under the controlled oxygen concentration (#3-1 to #3-3) or under the uncontrolled oxygen concentration (Comparative Example 1) to induce differentiation.

### BEST MODE FOR CARRYING OUT THE INVENTION

Hereinafter, embodiments of the present invention will be described in detail, but the following description is to facilitate understanding of the present invention. The scope of the present invention is not limited to the following embodiments, and other embodiments suitably substituted with features of the following embodiments by a person skilled in the art are also included in the scope of the present invention.

### [Explanation of Terminology]

In the present invention, "in the absence of an inhibitor" means "in a culture medium to which the inhibitor is not added".

In connection with the culture medium in the present invention, the term "is not added" indicates that a factor such as a protein, a peptide, or a compound specified as not added to a culture or a conditioned medium is not exogenously added. If a factor such as a protein, a peptide, or a compound specified as not added to a culture or a conditioned medium is brought in by continuous culture operation, the amount of the factor is adjusted to be less than 1 % (volume/volume), less than 0.5% (volume/volume), less than 0.1% (volume/volume), less than 0.05% (volume/volume), less than 0.01% (volume/volume), or less than 0.001% (volume/volume).

In connection with gene expression levels, the term "up-regulated" indicates that the expression of a gene is increased over that of a specific gene expression level in a cell population to be compared, and is, relative to the cell population to be compared, 1.1 times or more, 1.2 times or more, 1.3 times or more, 1.4 times or more, 1.5 times or more, 1.6 times or more, 1.7 times or more, 1.8 times or more, 1.9 times or more, 2.0 times or more, 2.1 times or more, 2.2 times or more, 2.3 times or more, 2.4 times or more, 2.5 times or more, 2.6 times or more, 2.7 times or more, 2.8 times or more, 2.9 times or more, 3.0 times or more, 3.1 times or more, 3.2 times or more, 3.3 times or more, 3.4 times or more, 3.5 times or more, 3.6 times or more, 3.7 times or more, 3.8 times or more, 3.9 times or more, 4.0 times or more, 4.1 times or more, 4.2 times or more, 4.3 times or more, 4.4 times or more, 4.5 times or more, 4.6 times or more, 4.7 times or more, 4.8 times or more, 4.9 times or more, 5.0 times or more, 5.1 times or more, 5.2 times or more, 5.3 times or more, 5.4 times or more, 5.5 times or more, 5.6 times or more, 5.7 times or more, 5.8 times or more, 5.9 times or more, 6.0 times or more, 6.1 times or more, 6.2 times or more, 6.3 times or more, 6.4 times or more, 6.5 times or more, 6.6 times or more, 6.7 times or more, 6.8 times or more, 6.9 times or more, 7.0 times or more, 7.1 times or more, 7.2 times or more, 7.3 times or more, 7.4 times or more, 7.5 times or more, 7.6 times or more, 7.7 times or more, 7.8 times or more, 7.9 times or more, 8.0 times or more, 8.1 times or more, 8.2 times or more, 8.3 times or more, 8.4 times or more, 8.5 times or more, 8.6 times or more, 8.7 times or more, 8.8 times or more, 8.9 times or more, 9.0 times or more, 9.1 times or more, 9.2 times or more, 9.3 times or more, 9.4 times or more, 9.5 times or more, 9.6 times or more, 9.7 times or more, 9.8 times or more, 9.9 times or more, 10.0 times or more, 20 times or more, 30 times or more, 40 times or more, 50 times or more, 60 times or more, 70 times or more, 80 times or more, 90 times or more, 100 times or more, 250 times or more, 500 times or more, 750 times or more, 1000 times or more, 5000 times or more, or 10000 times or more.

In connection with gene expression levels, the term "down-regulated" indicates that the expression of a gene is reduced from that of a specific gene expression level in a cell population to be compared, and is, relative to the cell population to be compared, 1.1 times or less, 1.2 times or less, 1.3 times or less, 1.4 times or less, 1.5 times or less, 1.6 times or less, 1.7 times or less, 1.8 times or less, 1.9 times or less, 2.0 times or less, 2.1 times or less, 2.2 times or less, 2.3 times or less, 2.4 times or less, 2.5 times or less, 2.6 times or less, 2.7 times or less, 2.8 times or less, 2.9 times or less, 3.0 times or less, 3.1 times or less, 3.2 times or less, 3.3 times or less, 3.4 times or less, 3.5 times or less, 3.6 times or less, 3.7 times or less, 3.8 times or less, 3.9 times or less, 4.0 times or less, 4.1 times or less, 4.2 times or less, 4.3 times or less, 4.4 times or less, 4.5 times or less, 4.6 times or less, 4.7 times or less, 4.8 times or less, 4.9 times or less, 5.0 times or less, 5.1 times or less, 5.2 times or less, 5.3 times or less, 5.4 times or less, 5.5 times or less, 5.6 times or less, 5.7 times or less, 5.8 times or less, 5.9 times or less, 6.0 times or less, 6.1 times or less, 6.2 times or less, 6.3 times or less, 6.4 times or less, 6.5 times or less, 6.6 times or less, 6.7 times or less, 6.8 times or less, 6.9 times or less, 7.0 times or less, 7.1 times or less, 7.2 times or less, 7.3 times or less, 7.4 times or less, 7.5 times or less, 7.6 times or less, 7.7 times or less, 7.8 times or less, 7.9 times or less, 8.0 times or less, 8.1 times or less, 8.2 times or less, 8.3 times or less, 8.4 times or less, 8.5 times or less, 8.6 times or less, 8.7 times or less, 8.8 times or less, 8.9 times or less, 9.0 times or less, 9.1 times or less, 9.2 times or less, 9.3 times or less, 9.4 times or less, 9.5 times or less, 9.6 times or less, 9.7 times or less, 9.8 times or less, 9.9 times or less, 10.0 times or less, 20 times or less, 30 times or less, 40 times or less, 50 times or less, 60 times or less, 70 times or less, 80 times or less, 90 times or less, 100 times or less, 250 times or less, 500 times or less, 750 times or less, 1000 times or less, 5000 times or less, or 10000 times or less.

### <Aggregate>

The aggregate in the present invention may be referred to alternatively by the term "clump", "cluster", or "spheroid", and generally refers to an assemblage of a group of cells that are not dissociated into single cells.

### <Pluripotent stem cell>

The pluripotent stem cells in the present invention refer to cells that have multilineage differentiation potential (pluripotency), being able to differentiate into all or multiple types of cells constituting a living body, and that can continue to proliferate endlessly while maintaining pluripotency in an in-vitro culture under suitable conditions. Specific examples thereof include embryonic stem cells (ES cells), pluripotent stem cells derived from embryonic primordial germ cells (EG cells; see Proc Natl Acad Sci USA, 1998, 95:13726-31), pluripotent stem cells derived from the spermary (GS cells; see Nature, 2008, 456:344-9), induced pluripotent stem cells (iPS cells), somatic stem cells (tissue stem cells), and the like. The pluripotent stem cells are preferably iPS cells or ES cells, and are more preferably iPS cells. The term "embryonic" refers to embryos derived by somatic nuclear transfer in addition to embryos derived by syngamy.

As the ES cells, it is possible to use cells derived from any warm-blooded animal, preferably a mammal. Examples of the mammals include mice, rats, guinea pigs, hamsters, rabbits, cats, dogs, sheep, swine, bovines, horses, goats, simians, and humans. Cells derived from humans are preferably used.

Specific examples of ES cells include ES cells of mammals or the like established by culturing early embryos before implantation, ES cells established by culturing early embryos produced by nuclear transfer of the nuclei of somatic cells, and ES cells obtained by modifying genes on chromosomes of these ES cells using genetic engineering techniques. The ES cells can be prepared in accordance with methods normally implemented in the relevant field and in publicly known documents. Mouse ES cells were established by Evans et al. (Evans et al., 1981, Nature 292:154-6) and Martin et al. (Martin, GR. et al., 1981, Proc Natl Acad Sci 78: 7634-8) in 1981. Human ES cells were established by Thomson et al. (Thomson et al., Science, 1998, 282:1145-7) in 1998, and are available from WiCell Research Institute (website: http://www.wicell.org/, Madison, Wisconsin, USA), the National Institute of Health in the USA, Kyoto University, the National Center for Child Health and Development, or the like, or can be purchased, for example, from Cellartis (website: http://www.cellartis.com/, Sweden).

Induced pluripotent stem cells (iPS cells) are cells having pluripotency, obtained by reprogramming somatic cells. Multiple groups have succeeded in producing iPS cells, including such groups as the group including professor Shinya Yamanaka at Kyoto University, the group including Rudolf Jaenisch at the Massachusetts Institute of Technology, the group including James Thomson at the University of Wisconsin, and the group including Konrad Hochedlinger at Harvard University. For example, the international patent publication WO 2007/069666 describes a somatic nucleus reprogramming factor containing the gene products of an Oct family gene, a Klf family gene, and a Myc family gene, as well as a somatic nucleus reprogramming factor containing the gene products of an Oct family gene, a Klf family gene, a Sox family gene, and a Myc family gene. The publication further describes a method for producing induced pluripotent stem cells by reprogramming somatic nuclei, comprising a step of bringing the above-mentioned nucleus reprogramming factors into contact with somatic cells.

The types of somatic cells used for producing the iPS cells are not particularly limited, and any type of somatic cell may be used. Specifically, the somatic cells include all cells constituting a living body other than reproductive cells, and may be differentiated somatic cells or undifferentiated stem cells. The somatic cells may be from any of mammals, birds, fish, reptiles, and amphibians, and there are no particular limitations. However, they are preferably from mammals (for example, rodents such as mice, or primates such as humans), and are particularly preferably from mice or humans. When human somatic cells are used, somatic cells from fetuses, newborns, or adults may be used. Specific examples of somatic cells include fibroblasts (for example, dermal fibroblasts), epithelial cells (for example, gastric epithelial cells, liver epithelial cells, and alveolar epithelial cells), endothelial cells (for example, blood vessels and lymph vessels), nerve cells (for example, neurons and glial cells), pancreatic cells, white blood cells (B cells, T cells, etc.), marrow cells, muscle cells (for example, skeletal muscle cells, smooth muscle cells, and cardiac muscle cells), hepatic parenchymal cells, nonhepatic parenchymal cells, adipose cells, osteoblasts, cells constituting the periodontium (for example, periodontal membrane cells, cementoblasts, gingival fibroblasts, and osteoblasts), and cells constituting the kidneys, the eyes and the ears.

iPS cells are stem cells having the ability to self-replicate over a long period of time under prescribed culture conditions (for example, under the conditions for culturing ES cells) and having multipotency for differentiation into any of ectodermal cells, mesodermal cells, or endodermal cells, under prescribed differentiation induction conditions. Additionally, when the iPS cells are transplanted to test animals such as mice, they may be stem cells having the ability to form teratomas.

To produce iPS cells from somatic cells, at least one or more reprogramming genes are first introduced into the somatic cells. The reprogramming genes are genes encoding reprogramming factors having the function of reprogramming the somatic cells so as to become iPS cells. Specific examples of combinations of reprogramming genes include, but are not limited to, the following combinations:
(i) an Oct gene, a Klf gene, a Sox gene, and a Myc gene;
(ii) an Oct gene, a Sox gene, a NANOG gene, and a L1N28 gene;
(iii) an Oct gene, a Klf gene, a Sox gene, a Myc gene, a hTERT gene, and a SV40 large T gene; or
(iv) an Oct gene, a Klf gene, and a Sox gene.

Aside from the above, a method in which transgenes are further reduced (Nature, 2008 Jul 31, 454(7204):646-50), a method using a low-molecular-weight compound (Cell Stem Cell, 2009 Jan 9, 4(1):16-9; Cell Stem Cell, 2009 Nov 6, 5(5):491-503), a method using transcription factor proteins instead of genes (Cell Stem Cell, 2009 May 8, 4(5):381-4), and the like have been reported, and the iPS cells may be iPS cells produced by any of these methods.

Although the mode of introduction of the reprogramming factors into cells is not particularly limited, examples include gene transfer using plasmids, transfection with synthetic RNA, and direct introduction of the proteins. Additionally, iPS cells produced by methods using microRNA or RNA, low-molecular-weight compounds, or the like may be used. The pluripotent stem cells, including ES cells and iPS cells, may be commercially available products or cells received by distribution, or may be newly produced.

As the iPS cells, it is possible to use, for example, the 253G1 cell line, the 253G4 cell line, the 201B6 cell line, the 201B7 cell line, the 409B2 cell line, the 454E2 cell line, the 606A1 cell line, the 610B1 cell line, the 648A1 cell line, the 1201C1 cell line, the 1205D1 cell line, 1210B2 cell line, the 1231A3 cell line, the 1383D2 cell line, the 1383D6 cell line, the iPS-TIG120-3f7 cell line, the iPS-TIG120-4f1 cell line, the iPS-TIG114-4f1 cell line, the RPChiPS771-2 cell line, the 15M63 cell line, the 15M66 cell line, the HiPS-RIKEN-1A cell line, the HiPS-RIKEN-2A cell line, the HiPS-RIKEN-12A cell line, the Nips-B2 cell line, the TkDN4-M cell line, the TkDA3-1 cell line, the TkDA3-2 cell line, the TkDA3-4 cell line, the TkDA3-5 cell line, the TkDA3-9 cell line, the TkDA3-20 cell line, the hiPSC 38-2 cell line, the MSC-iPSC1 cell line, the BJ-iPSC1 cell line, and the like.

As the ES cells, it is possible to use, for example, the KhES-1 cell line, the KhES-2 cell line, the KhES-3 cell line, the KhES-4 cell line, the KhES-5 cell line, the SEES1 cell line, the SEES2 cell line, the SEES3 cell line, the HUES8 cell line, the CyT49 cell line, the H1 cell line, the H9 cell line, the HS-181 cell line, and the like. Newly produced clinical-grade iPS cells or ES cells may also be used.

### <Endodermal cell>

Endodermal cells have the ability to differentiate into the tissues of organs such as the digestive tract, the lung, the thyroid gland, the pancreas, and the liver, the cells of secretory glands opening onto the digestive tract, and the peritoneum, the pleura, the larynx, the auditory tube, the trachea, the bronchi, and the urinary tract (most of the bladder and the urethra, and part of the ureter). In general, they are sometimes referred to as the definitive endoderm (DE). Differentiation from pluripotent stem cells to endodermal cells can be confirmed by measuring the expression levels of genes specific to endodermal cells. Examples of genes specific to endodermal cells include SOX17, FOXA2, CXCR4, AFP, GATA4, HOMES, and the like. In the present description, endodermal cells are sometimes referred to alternatively as the definitive endoderm.

### <Primitive gut tube cell>

Primitive gut tube cells form the foregut, the midgut, and the hindgut. The midgut is connected to the yolk sac and the extraembryonic allantois branches from the hindgut. Additionally, the pharynx in the respiratory system is also formed from the foregut. There are organs, such as the stomach and the intestines, into which the gut tubes directly differentiate, and those like the liver, the gall bladder, the pancreas, (the spleen (lymphoid organs)), and the like that are formed by budding from gut tubes. The differentiation from endodermal cells to primitive gut tube cells can be confirmed by measuring the expression levels of genes that are specific to primitive gut tube cells. Examples of genes that are specific to primitive gut tube cells include HNF-1β, HNF-4α, and the like.

### <Posterior foregut (PFG) cell>

The differentiation from primitive gut tube cells to posterior foregut cells can be confirmed by measuring the expression levels of genes specific to posterior foregut cells. Examples of genes specific to posterior foregut cells include PDX1, HNF6, and the like.

### <Pancreatic progenitor (PP) cell>

Pancreatic progenitor cells are cells that are differentiated from posterior foregut cells, the cells being able to differentiate to pancreatic exocrine cells and endocrine cells. The differentiation from posterior foregut cells to pancreatic progenitor cells can be confirmed by measuring the expression levels of genes specific to pancreatic progenitor cells. Examples of genes specific to pancreatic progenitor cells include PDX1, NKX6.1, and the like.

### <Pancreatic endocrine precursor (EP) cell>

Pancreatic endocrine precursor cells are cells that are differentiated from pancreatic progenitor cells, the cells being able to differentiate to pancreatic endocrine cells (α cells, β cells, δ cells, ε cells, PP cells, etc.). The differentiation into pancreatic endocrine precursor cells can be confirmed by measuring the expression levels of genes specific to pancreatic endocrine precursor cells. Examples of genes specific to pancreatic endocrine precursor cells include PDX1, NKX6.1, NeuroG3, NeuroD1, and the like.

### <Pancreatic α cell>

Pancreatic α cells are cells that are differentiated from pancreatic endocrine precursor cells, the cells secreting glucagon. The differentiation from pancreatic endocrine precursor cells to pancreatic α cells can be confirmed by measuring the expression levels of genes specific to pancreatic α cells. Examples of genes specific to pancreatic α cells include glucagon (GCG), ARK, IRX2, GC, TM4SF4, TTR, CRYBA2, and the like.

### <Pancreatic β cell>

Pancreatic β cells are cells that are differentiated from pancreatic endocrine precursor cells, the cells secreting insulin. The differentiation from pancreatic endocrine precursor cells to pancreatic β cells can be confirmed by measuring the expression levels of genes specific to pancreatic β cells. Examples of genes specific to pancreatic β cells include insulin, NKX6.1, MAFA, PDX1, and the like.

### <Signaling and factors>

### (Bone morphogenetic protein (BMP) signaling inhibitor)

Bone morphogenetic protein (BMP) signals are signals that are mediated by bone morphogenetic protein (BMP) ligands, serving various roles in vertebrates. During embryogenesis, the dorsoventral axis is established by a BMP signaling gradient formed by the coordinated expression of ligands, receptors, coreceptors, and soluble antagonists. BMP is a regulator that is important for gastrulation, mesodermal induction, organogenesis, and cartilaginous bone formation, and that controls the fates of pluripotent stem cell populations.

BMP receptors comprise complexes of type I receptors (activin receptor-like kinase; ALK-1, ALK-2, ALK-3 or ALK-6) and type II receptors (ActRII, ActRIIB or BMPRII), and the activated type I receptor kinases cause phosphorylation of two serine residues located on the C terminus of the R-Smad (receptor-regulated Smad) protein. An R-Smad (Smad1, Smad5 or Smad8) that is phosphorylated by the ligand (BMP) binding to a receptor is called a BR-Smad (BMP R-Smad). Two molecules of R-Smad that have been phosphorylated form a heterotrimer with Smad4 and undergo nuclear translocation, thereby regulating the transcription of target genes.

The bone morphogenetic protein (BMP) signaling inhibitor is not particularly limited as long as it is a substance that inhibits BMP signaling, which begins with ligands (BMP-4 or the like) binding to receptors. However, it is preferably a substance that inhibits at least one of ALK-1, ALK-2, ALK-3, and ALK-6. Additionally, a substance that inhibits a ligand binding to a receptor (such as an antagonist antibody) may be used as the BMP signaling inhibitor.

The bone morphogenetic protein (BMP) signaling inhibitor is not particularly limited, but inhibitors and the like that act on type I receptors (ALK-1, ALK-2, ALK-3, or ALK-6) can effectively inhibit BMP signaling, and examples include dorsomorphin, LDN193189, LDN-214117, LDN-212854, K02288, ML347, and the like.

### (Retinoic acid)

Retinoic acid is a carboxylic acid derivative of vitamin A, and exists in the form of several stereoisomers such as all-trans retinoic acid (also known as tretinoin), 9-cis retinoic acid (also known as alitretinoin), and 13-cis retinoic acid (also known as isotretinoin). Retinoic acid serves the main role in the bioactivity of retinoids and carotenoids in the living body, as a natural ligand of retinoic acid receptor (RAR), which is one of nuclear receptors. RAR is known to form a heterodimer with retinoid X receptor (RXR, the ligand is 9-cis retinoic acid) and serve as a ligand-inducible transcription factor that binds to promoters in specific target gene groups, thereby positively or negatively controlling the expression of the target gene groups by the transcription level. Even compounds having chemical structures that are not at all similar to vitamin A are referred to as retinoids, including synthetic compounds exhibiting extremely high binding affinity to these specific receptors.

### (Retinoic acid analog)

Retinoic acid (RA) is known to have the function of promoting cell differentiation and apoptosis, or stopping the cell division cycle, and is also used when inducing the differentiation of pluripotent stem cells. The retinoic acid analog is not limited as long as it is a substance that activates retinoic acid receptor (PAR) and retinoid X receptor (RXR), which are nuclear receptors. Examples include EC23, EC19, AC 261066, AC 55649, adapalene, AM 580, AM 80, BMS 753, BMS 961, CD 1530, CD 2314, CD 437, Ch 55, isotretinoin, tazarotene, TTNPB, and the like. EC23 is a stable compound that is less susceptible to degradation by light than retinoic acid is, and can therefore be favorably used.

### (Ascorbic acid)

Ascorbic acid, known as vitamin C which is a water-soluble vitamin, is an organic compound having a lactone structure. Ascorbic acid is an optically active compound. There are L-form and D-form, but L-form is preferable. One that is known as vitamin C is the L form. Ascorbic acid has a strong reduction power and is involved in various redox reactions in a living body. Ascorbic acid is absorbed from the upper part of the small intestine and partially metabolized in the liver via the portal vein. However, most of it is taken up by tissues of the adrenal gland, the pituitary gland, and the like. Ascorbic acid is essential for inhibiting the generation of peroxides, detoxifying xenobiotic substances, metabolizing tyrosine, and promoting absorption of non-heme iron. In addition, it is involved in hydroxylation in reactions such as biosynthesis of collagens, catecholamines, and carnitine, and cholesterol metabolism.

### (ROCK signaling inhibitor)

ROCK (rho-associated coiled-coil-forming kinase/rho-binding kinase) converts myosin light chains to an activated structure by means of phosphorylation of the myosin light chains and myosin light chain phosphatase. Furthermore, ROCK deactivates cofilin, which is an actin-depolymerizing factor, by phosphorylation of LIM kinase, thus suppressing actin depolymerization. In addition thereto, ROCK phosphorylates many substrates and is involved in diverse biological functions such as cell motility, cell polarity, cell adhesion, cell division, apoptosis, and transcriptional regulation. Examples of ROCK signaling inhibitors include Y27632, thiazovivin, fasudil (HA-1077) HCl, GSK429286A, RKI-1447, GSK180736A (GSK1 80736), hydroxyfasudil (HA-1100) HCl, Y-39983 HCl, netarsudil (AR-13324) 2HCl, GSK269962 A HCl, Ripasudil (K-115) hydrochloride dihydrate, KD025 (SLx-2119), AT13148, and the like.

### (TGFβ superfamily signaling activator)

TGFβ superfamily signaling plays a very important role in the regulation of cell proliferation, differentiation, and the development of a wide variety of biological systems. In general, signaling is initiated by serine/threonine receptor kinase multimer formation caused by ligands, and by the phosphorylation of intracellular signaling molecules such as Smad1/5/8 for the bone morphogenetic protein (BMP) pathway, or by the phosphorylation of Smad2/3 for the TGFβ/activin pathway and the NODAL/activin pathway. The phosphorylation of the carboxyl group terminals of Smads by activated receptors results in the formation of partners with Smad4, which is a signal transducer similar thereto, promoting nuclear translocation. It is known that activated Smads control various biological effects by partnering with transcription factors to perform transcriptional regulation that is specific to the cell state.

Examples of genes involved in the TGFβ superfamily signaling pathway include the activin A gene, the BMP2 gene, the BMP3 gene, the BMP4 gene, the BMP5 gene, the BMP6 gene, the BMP7 gene, the BMP8 gene, the BMP13 gene, the GDF2 (growth differentiation factor 2) gene, the GDF3 gene, the GDF5 gene, the GDF6 gene, the GDF7 gene, the GDF8 gene, the GDF11 gene, the TGF-β1 gene, the TGF-β2 gene, the TGF-β3 gene, the AMH (anti-Mullerian hormone) gene, the paired-like homeodomain 2 (PITX2) gene, the NODAL gene, and the like.

The TGFβ superfamily signaling activator is not particularly limited as long as it is a substance that activates signaling on the bone morphogenetic protein (BMP) pathway, the TGFβ/activin pathway, and/or the NODAL/activin pathway. For example, it is possible to use activin A, BMP2, BMP3, BMP4, BMP5, BMP6, BMP7, BMP8, BMP13, GDF2, GDF5, GDF6, GDF7, GDF8, GDF11, TGF-β1, TGF-β2, TGF-β3, AMH, PITX2, and/or NODAL. In particular, a substance that activates signaling on the TGFβ/activin pathway can be favorably used. Specifically, it is preferable to use at least one type selected from the group consisting of activin A and BMP4, and it is particularly preferable to use activin A and BMP4.

### (Protein kinase C (PKC) activator)

PKC is a protein associated with the control of many cell functions such as cell proliferation and cell death, gene transcription and translation, cell morphology, intercellular contact, and the like. PKC is a type of protein kinase that phosphorylates hydroxyl groups in serine residues and threonine residues in matrix proteins, and there are at least ten or more isozymes thereof. The isozymes are classified in three subfamilies, namely, classical, novel, and atypical, depending on the structures, the activation mechanisms, and the bioactivity thereof. The classical PKC isozymes (α, βI, βII, and γ) require diacyl glycerol (DAG) and Ca²⁺ for the activation thereof. DAG is generated together with inositol 1,4,5-triphosphate (1P3) by the hydrolysis of phosphatidyl inositol 4,5-diphosphate (PIP2) by means of phospholipase C (PLC). When IP3 disperses in a cell and binds to an IP3-sensitive Ca²⁺ channel in the endoplasmic reticulum, the Ca²⁺ ions are released into the cytoplasm. When these Ca²⁺ ions bind with PKC, the PKC moves into the cell membrane. There, PKC interacts with DAG at the C1 domain. Then, the PKC undergoes a structural change in which the regulatory region is separated from the catalytic domain thereof, and thus becomes active. The novel PKC isozymes (δ, ε, θ, and η) are activated only by DAG. This is due to the fact that the affinity of the C1 domain in novel PKC isozymes to DAG is much higher than that of the classical types. The atypical PKC isozymes (ζ, Mζ, and ι/λ) do not require either DAG or Ca²⁺ for the activation thereof, and are activated by means of second messengers that are various lipid metabolism products (Khalil, 2010; Wu-Zhang and Newton, 2013; Mochly-Rosen et al., 2012).

The PKC activator may be any substance that activates at least one or more PKC isozymes among the above-mentioned PKC isozymes.

Although the PKC activator is not particularly limited, it is possible to use, for example, indolactam V (ILV), okadaic acid, phorbol-12-myristate 13-acetate (PMA), bryostatin 1, 1alpha,25-dihydroxyvitamin D3, prostratin, 1,2-dioctanoyl-sn-glycerol, 1-oleoyl-2-acetyl-sn-glycerol (OAG), oleic acid, ingenol 3-angelate, DCP-LA, PIP2, phorbol-12,13-dibutyrate, 8(S)-HETE, and derivatives thereof.

### (Notch signaling inhibitor)

Notch signaling is highly involved in the survival, the proliferation and the differentiation of many cells, and is also involved in ontogeny together with cytokine/tyrosine kinase, Wnt, the TGF-β family/Smad, hedgehog, and integrin. Abnormalities in Notch signaling are also associated with carcinogenesis, the proliferation of cancer cells, and the survival of cancer stem cells. Notch receptor is a single-pass transmembrane protein that comprises a functional extracellular domain (NECD), a transmembrane domain (TM), and an intracellular domain (NICD). As a result of processing of Notch receptor in the endoplasmic reticulum (ER) and the Golgi body in a cell that receives a signal, cleavage occurs (S1 cleavage), and furthermore, a glycan binds thereto, generating a heterodimer that is stabilized by calcium ions (Ca²⁺), but this is formed from the NECD, which is attached by non-covalently bonding to the TM-NICD inserted in the membrane. The receptor that has been processed in this way is next transferred to the cell membrane, and it becomes possible for a ligand to bind thereto. In mammals, members of the Delta-like (DLL1, DLL3, and DLL4) and Jagged (JAG1 and JAG2) families, which are present in the cells sending the signals, function as ligands for the Notch signal receptors. When a ligand binds thereto, the NECD is immediately cleaved (S2 cleavage) from the TM-NICD domain by TACE (ADAM metal protease TNF-α converting enzyme). This NECD keeps the ligand in a bound state, and this complex undergoes endocytosis and recycling/degradation in the cell on the signaltransmitting side. In the cell on the signal-receiving side, γ-secretase (also involved in Alzheimer's disease) releases (S3 cleavage) the NICD from the TM, as a result of which the NICD is translocated into the nucleus, where it is associated with a CSL (CBF1/Su(H)/Lag-1) family transcription factor complex. As a result thereof, Myc, p21, and HES family members, which are standard Notch target genes, are subsequently activated.

Examples of the Notch signaling inhibitor include IMR-1, FLI-06, crenigacestat (LY3039478), and the like. Additionally, γ-secretase inhibitors such as DBZ (dibenzazepine), DAPT (N-[N-(3,5-difluorophenacetyl-L-alanyl)]-(S)-phenylglycine t-butyl ester), LY411575, dibenzazepine (YO-01027), RO4929097, nirogacestat (PF-03084014, PF-3084014), L-685,458, semagacestat (LY450139), avagacestat (BMS-708163), MK-0752, and the like may also be used as the Notch signaling inhibitor.

### (Hedgehog (HH) signaling inhibitor)

Hedgehog (HH) signals are known as being embryonic cell growth factors and morphogenetic factors. Additionally, they have been demonstrated as being capable of functioning to control tissue stem cells as well as homeostasis and tissue regeneration in adults. Abnormalities in embryonic HH signaling are a cause of congenital diseases such as holoprosencephaly, and the sustained activity of HH signaling in adults is considered to be associated with various forms of cancer including skin basal cell carcinoma and medulloblastoma. As hedgehog signaling ligands, three types of HH ligands (SHH, Sonic hedgehog; IHH, Indian hedgehog; and DHH, Desert hedgehog) are known in mammals. In the state in which there are no hedgehog ligands (the off state), Patched, which is a receptor of the hedgehog family ligands, normally binds to Smoothened (Smo), which is a G protein-coupled transmembrane protein, and inhibits the association of Smoothened with the membrane. In the off state, SuFu and COS2 (which is Kif7 in vertebrates) isolate groups of Gli, which is a transcription factor that binds to microtubules, in the primary cilium. Gli is phosphorylated by PKA, CKI and GSK-3, and Gli activating factors (Gli1 and Gli2 in mammals) are decomposed by β-TrCP, or Gli suppression factors (Gli3 or truncated Ci in Drosophila) are produced in a preserved pathway, which leads to suppression of hedgehog target genes. In the activated state (the on state), the hedgehog ligand binds to Patched, thereby allowing Smoothened, mediated by β-arrestin, to move into the primary cilium, where the activity of G proteins associated therewith inhibits the inhibitory kinase activity that acts on Gli, allowing Gli to freely undergo nuclear translocation, thereby activating hedgehog target genes such as those for Cyclin D, Cyclin E, Myc, and Patched.

The hedgehog (HH) signaling inhibitor is not particularly limited as long as it is a substance that inhibits the above-mentioned hedgehog signaling, but examples thereof are substances that inhibit signaling by acting on Smo and the like. Additionally, antagonist antibodies that inhibit the binding of the hedgehog ligands to receptors such as Patched may also be used as the hedgehog signaling inhibitor.

The hedgehog (HH) signaling inhibitor is not particularly limited, but examples include SANT1, cyclopamine, sonidegib, PF-5274857, glasdegib, taladegib, BMS-833923, MK-4101, vismodegib, GANT61, jervine, HPI-4, and the like. For example, SANT1 is an HH signaling antagonist that has strong cell penetration properties and that inhibits signaling by binding directly to the Smo receptors, and thus can be favorably used.

### (TGF-β receptor signaling inhibitor)

TGF-β receptor (TGFβ) signaling is signaling that involves ligands of transforming growth factor β (TGFβ), and that plays a central role in cell processes such as, for example, the growth, proliferation, differentiation and apoptosis of cells. The binding of TGFβ ligands to type II receptors (serine/threonine kinase), which gradually increases and phosphorylates type I receptors (ALK5), is involved in TGFβ signaling. Next, these type I receptors phosphorylate receptor-regulated SMADs (R-SMADs; for example, SMAD1, SMAD2, SMAD3, SMAD5, SMAD8, or SMAD9) that bind to SMAD4. Then, these SMAD complexes enter nuclei and serve roles in transcriptional regulation.

The TGFβ signaling inhibitor is not particularly limited as long as it is a substance that inhibits the above-mentioned TGFβ signaling, but examples thereof are substances that act on ALK5 and inhibit the phosphorylation thereof. Additionally, antagonist antibodies that inhibit the binding of TGFβ to receptors and the like may also be used as the TGFβ signaling inhibitor.

The TGFβ signaling inhibitor is not particularly limited, but for example, inhibitors that act on ALK5 can be favorably used. Examples include SB431542, galunisertib, LY2109761, SB525334, SB505124, GW788388, LY364947, RepSox, SD-208, vactosertib, LDN-212854, and the like.

### (Insulin receptor signaling activator)

Insulin receptors are expressed in the liver, skeletal muscles, adipose tissue, nerve cells, and the like, and insulin receptor signaling is known to be involved in the formation, maintenance and repair of the neural network. Insulin is an important hormone that regulates important energy functions such as glucose and lipid metabolism. Insulin activates insulin receptor (IR) tyrosine kinase and performs recruitment and phosphorylation of different substrate adapters such as the IRS (insulin receptor substrate) family. Tyrosine-phosphorylated IRS provides binding sites to many signaling partners. Among these, PI3K (phosphoinositide 3-kinase) plays an important role in insulin function, mainly through the activation of Akt (protein kinase B) and PKC (protein kinase C). Activated Akt causes glycogen synthesis by inhibiting GSK-3 (glycogen synthase kinase), protein synthesis by means of mTOR (mammalian target of rapa) and downstream factors, and cell survival by inhibiting proapoptotic factors (Bad, transcription factor Forkhead family, GSK-3, etc.). Insulin receptor signaling also has cell growth and cell division effects, and as with the activation of the Ras/MAPK pathway, Akt cascades are mainly involved in the effects thereof.

Although the insulin receptor signaling activator is not particularly limited as long as it is a substance that activates the above-mentioned insulin receptor signaling, examples include ligands that bind to insulin receptor and IGF receptor. Additionally, it may be a substance that directly or indirectly activates PI3K, PKC or Akt.

The insulin receptor signaling activator is preferably insulin, insulin-like growth factor-1 (IGF-1), IGF-2, or the like. Additionally, PI3-kinase activator (Santa Cruz, product number sc-3036), 740 Y-P and the like, which are PI3K activators, can also be used as the insulin receptor signaling activator.

### (FGF receptor signaling activator)

FGF (fibroblast growth factor) receptor signaling is signaling that is mediated by FGF receptors and that occurs on the RAS-MAPK pathway and the PI3K-AKT pathway. It is involved in various cell functions such as cell proliferation, cell death, angiogenesis, epithelial-to-mesenchymal transitions (EMT), and the like, and also serves an important role in controlling embryogenesis and post-natal development of the skeletal structure.

It is sufficient for the FGF receptor signaling activator to be a substance that activates signaling as mentioned above, and typical examples thereof are ligands (FGF family) that bind to FGF receptors. Additionally, activators of the RAS-MAPK pathway and the PI3K-AKT pathway may also be used as FGF receptor signaling activators.

Examples of FGF receptor signaling activators include those in the FGF family, among which FGF7, FGF3, FGF10, FGF22, FGF1, FGF2, FGF4, FGF5, FGF6, FGF8, FGF17, FGF18, FGF9, FGF16, FGF20, FGF19, FGF21, FGF23, and the like are preferable, and FGF7 is particularly preferable.

### (EGF receptor signaling activator)

Epidermal growth factor (EGF) is a 6045 Da protein, composed of 53 amino acid residues and three intramolecular disulfide bonds, which binds as a ligand to epidermal growth factor receptor (EGFR) that is present on cell surfaces, and which serves an important role in regulating cell growth and proliferation. Examples of EGF receptor signaling activators include EGF and the like.

### (GLP-1 (glucagon-like peptide-1) receptor signaling activator)

Glucagon-like peptide-1 (GLP-1), which is an incretin hormone that is secreted in the living body, increases cAMP by acting through GLP-1 receptor, and promotes insulin secretion in a manner dependent on the glucose concentration. Examples of GLP-1 receptor signaling activators include GLP-1, Exendin 4, and the like.

### (Growth factor stabilizer)

Growth factor stabilizers refer to substances having a growth factor stabilization function. Growth factor stabilization is not particularly limited as long as it is a function for suppressing the reduction of the amount of extracellular growth factors, such as the function of suppressing the decomposition of growth factors or the function of promoting the extracellular secretion of growth factors. Examples of growth factor stabilizers include heparin, heparan sulfate, and the like.

### (Hepatocyte growth factor)

Hepatocyte growth factor (HGF) is a type of cytokine that is involved in tissue regeneration and repair in the living body. Hepatocyte growth factor is known to act on various cells besides hepatocytes, promoting the proliferation of epithelial cells and endothelial cells, and acting as a neurotrophic factor on nerve cells.

### (Insulin-like growth factor)

Insulin-like growth factor is a polypeptide having a structure similar to proinsulin, and is known to be involved in cell proliferation, survival and migration, and in the production of the extracellular matrix including collagen. Insulin-like growth factor exhibits cell division promotion activity by binding to a common receptor known as insulin-like growth factor receptor-1 (IGFR1) in various cell types including tumor cells. Examples of insulin-like growth factors include insulin-like growth factor-I (1GF-I) and insulin-like growth factor-II (IGF-II).

### (Adenylate cyclase activator (substance elevating intracellular cAMP concentration))

Adenylate cyclase is an enzyme that catalyzes the conversion of ATP to 3',5'-cycloAMP (cAMP) and pyrophosphate. cAMP is a molecule, known as a second messenger, that is important for signaling in eukaryotes. Examples of adenylate cyclase activators include forskolin and NKH477.

### (WNT signaling activator)

WNT signaling refers to a series of actions to promote nuclear translocation of β-catenin, and to activate the functions thereof as a transcription factor. WNT signaling is caused by intercellular interactions, and for example, includes a series of processes in which a protein known as WNT3A, secreted from a certain cell, acts on another cell, causing β-catenin in the cell to undergo nuclear translocation and to act as a transcription factor. The series of processes triggers the first phenomena of organ construction, such as epithelial-mesenchymal interactions. WNT signaling is known to control various cell functions including proliferation and differentiation of cells, and cell motility in organogenesis and early development, by the activation of three pathways, namely, the β-catenin pathway, the PCP pathway, and the Ca²⁺ pathway.

Examples of genes involved in the WNT signaling pathway include the WNT3A gene and the like.

The WNT signaling activator is not particularly limited, and may be of any type as long as it exhibits inhibitory activity against glycogen synthase kinase-3 (GSK-3). It is possible to use, for example, a bis-indolo (indirubin) compound (BIO) ((2'Z,3'E)-6-bromoindirubin-3'-oxime), an acetoxime analog thereof, namely, BIO-acetoxime ((2'Z,3'E)-6-bromoindirubin-3'-acetoxime), a thiadiazolidine (TDZD) analog (4-benzyl-2-methyl-1,2,4-thiadiazolidine-3,5-dione), an oxothiadiazolidine- 3-thione analog (2,4-dibenzyl-5-oxothiadiazolidine-3-thione), a thienyl α-chloromethyl ketone compound (2-chloro-1-(4,4-dibromo-thiophen-2-yl)-ethanone), a phenyl α-bromomethyl ketone compound (α-4-dibromoacetophenone), a thiazole-containing urea compound (N-(4-methoxybenzyl)-N'-(5-nitro-1,3-thiazol-2-yl)urea), a GSK-3β peptide inhibitor such as H-KEAPPAPPQSpP-NH₂, particularly preferably CHIR99021 (CAS: 252917-06-9), or the like. WNT3A can also be favorably used.

### [1] Method for inducing differentiation into pancreatic α cells

A method for inducing differentiation into pancreatic α cells of the present invention is a method including: a step of inducing differentiation from endodermal cells, which have been induced to differentiate from pluripotent stem cells, into primitive gut tube (PGT) cells; a step of inducing differentiation from the primitive gut tube (PGT) cells into pancreatic endocrine precursor (EP) cells; and a step of inducing differentiation from the pancreatic endocrine precursor cells into pancreatic α cells. According to the method for inducing differentiation into pancreatic α cells of the present invention, pancreatic α cells can be produced from pluripotent stem cells.

A method for inducing differentiation from endodermal cells, which have been induced to differentiate from pluripotent stem cells, into pancreatic α cells will be described.

In the step (a) of the present invention, endodermal cells, which have been induced to differentiate from pluripotent stem cells, are cultured in the presence of a bone morphogenetic protein (BMP) signaling inhibitor, and retinoic acid or a retinoic acid analog to induce differentiation into primitive gut tube (PGT) cells.

In the step (b) of the present invention, the primitive gut tube (PGT) cells are cultured to be induced to differentiate into pancreatic endocrine precursor (EP) cells.

In the step (c) of the present invention, the pancreatic endocrine precursor (EP) cells are cultured to be induced to differentiate into pancreatic α cells.

The step (b) and the step (c) are performed in the absence of ascorbic acid. Hereinafter, each step will be explained. The induction of differentiation from pluripotent stem cells into endodermal cells will be described below.

### <Culture conditions>

The culturing of the cells when inducing the differentiation from endodermal cells into pancreatic α cells may be implemented by using either an adhesion culture or a suspension culture, but a suspension culture is preferable. The cells may be suspension cultured by being adhered to a microcarrier or the like or may be suspension cultured in the form of cell clumps composed only of cells, or a polymer such as collagen may be intermixed into the cell clumps. Thus, the form of the culture is not particularly limited.

The culture temperature when inducing differentiation from the endodermal cells to pancreatic α cells is not particularly limited as long as the culture temperature is suitable for culturing the cells that are used, but the culture temperature should generally be 30°C to 40°C, preferably approximately 37°C.

The cells are preferably cultured by using a CO₂ incubator or the like in an atmosphere with a CO₂ concentration of about 1% to 10%, preferably 5%.

### <Culture period>

The culture period for inducing differentiation from the endodermal cells into the primitive gut tube (PGT) cells in the step (a) is generally from 24 hours to 120 hours, and is preferably from about 48 hours to 96 hours. For example, it may be 72 hours.

The induction of differentiation from the primitive gut tube (PGT) cells into the pancreatic endocrine precursor (EP) cells in the step (b) includes the induction of differentiation from the primitive gut tube (PGT) cells into posterior foregut (PFG) cells, the induction of differentiation from the posterior foregut (PFG) cells into pancreatic progenitor (PP) cells, and the induction of differentiation from the pancreatic progenitor (PP) cells into pancreatic endocrine precursor (EP) cells.

The culture period for the differentiation culture from the primitive gut tube (PGT) cells into the posterior foregut (PFG) cells is not particularly limited as long as the cells are converted to a cell type in which the cell properties of posterior foregut (PFG) cells are exhibited. For example, it is sufficient for the period to be 2 weeks or less, more specifically 1 day or more and 10 days or less, more preferably 2 days or more and 7 days or less, even more preferably 3 days or more and 5 days or less, and in one example, 4 days.

The culture period for the differentiation culture from the posterior foregut (PFG) cells into the pancreatic progenitor (PP) cells is not particularly limited as long as the cells are converted to a cell type in which the cell properties of pancreatic progenitor (PP) cells are exhibited. For example, it is sufficient for the period to be 2 weeks or less, more specifically 1 day or more and 10 days or less, more preferably 2 days or more and 7 days or less, even more preferably 2 days or more and 4 days or less, and in one example, 3 days.

The culture period for the differentiation culture from the pancreatic progenitor (PP) cells into the pancreatic endocrine precursor (EP) cells is not particularly limited as long as the cells are converted to a cell type in which the cell properties of pancreatic endocrine precursor (EP) cells are exhibited. For example, it is sufficient for the period to be 2 weeks or less, more specifically 1 day or more and 10 days or less, more preferably 3 days or more and 10 days or less, even more preferably 5 days or more and 9 days or less, and in one example, 7 days.

The culture period for the differentiation culture into pancreatic α cells in the step (c) is not particularly limited as long as the cells are converted to a cell type in which the cell properties of pancreatic α cells are exhibited. For example, it is sufficient for the period to be 3 weeks or less, more specifically 3 days or more and 20 days or less, more preferably 5 days or more and 14 days or less, even more preferably 7 days or more and 12 days or less, and in one example, 10 days.

### <Culture medium>

As the culture medium used in the step (a) to the step (c), an MEM medium, a BME medium, a DMEM medium, a DMEM/F12 medium, an αMEM medium, an IMDM medium, an ES medium, a DM-160 medium, a Fisher medium, an F12 medium, a WE medium, an RPM11640 medium, an Essential 6^{™} medium (Thermo Fisher Scientific), a culture medium in which two or more culture media arbitrarily selected from these culture media are mixed, or the like may be used in accordance with the types of cells. The above-mentioned culture media may include glucose, the glucose concentration being preferably 1 mM or more and 100 mM or less, more preferably 2 mM or more and 50 mM or less, and even more preferably 5 mM or more and 30 mM or less.

The culture medium may further contain bovine serum albumin (BSA) or human serum albumin (HSA). Preferably, the BSA or HSA contains 2 mg/g or less of lipids and 0.2 mg/g or less of free fatty acids.

The lower limit of the amount of BSA added to the culture medium is preferably 0.01%, more preferably 0.05%, more preferably 0.10%, more preferably 0.15%, more preferably 0.20%, and more preferably 0.25%. The upper limit of the amount of BSA added to the culture medium is preferably 1.00%, more preferably 0.90%, more preferably 0.80%, more preferably 0.70%, more preferably 0.60%, more preferably 0.50%, more preferably 0.40%, more preferably 0.30%, and more preferably 0.25%.

The culture medium may further contain antibiotics such as penicillin and streptomycin. For example, the culture medium may include 0.1 % to 2% (volume/volume) of penicillin and 0.1% to 2% (volume/volume) of streptomycin.

The culture medium may also contain a B27 (registered trademark) supplement. The lower limit of the amount of the B27 (registered trademark) supplement added to the culture medium is preferably 0.01%, more preferably 0.1%, more preferably 0.2%, more preferably 0.3%, more preferably 0.4%, more preferably 0.5%, more preferably 0.6%, more preferably 0.7%, more preferably 0.8%, and more preferably 0.9%. The upper limit of the amount of the B27 (registered trademark) supplement added to the culture medium is preferably 10%, more preferably 9%, more preferably 8%, more preferably 7%, more preferably 6%, more preferably 5%, more preferably 4%, more preferably 3%, more preferably 2%, and more preferably 1%.

The culture medium may contain insulin, transferrin, and selenous acid. The insulin, transferrin, and selenous acid may be contained in the culture medium in the form of a commercially available mixture such as a B27 supplement.

The lower limit of the amount of transferrin added to the culture medium is preferably 0.001 mg/L, more preferably 0.01 mg/L, more preferably 0.1 mg/L, more preferably 1 mg/L, more preferably 1.1 mg/L, more preferably 1.2 mg/L, more preferably 1.3 mg/L, and more preferably 1.4 mg/L. The upper limit of the amount of transferrin added to the culture medium is preferably 1000 mg/L, more preferably 500 mg/L, more preferably 100 mg/L, more preferably 90 mg/L, more preferably 80 mg/L, more preferably 70 mg/L, more preferably 60 mg/L, more preferably 50 mg/L, more preferably 40 mg/L, more preferably 30 mg/L, more preferably 20 mg/L, more preferably 10 mg/L, more preferably 9 mg/L, more preferably 8 mg/L, more preferably 7 mg/L, more preferably 6 mg/L, more preferably 5 mg/L, more preferably 4 mg/L, and more preferably 3 mg/L.

The lower limit of the amount of selenous acid added to the culture medium is preferably 0.001 µg/L, more preferably 0.01 µg/L, more preferably 0.1 µg/L, more preferably 1 µg/L, more preferably 1.1 µg/L, more preferably 1.2 µg/L, more preferably 1.3 µg/L, more preferably 1.4 µg/L, more preferably 1.5 µg/L, more preferably 1.6 µg/L, and more preferably 1.7 µg/L. The upper limit of the amount of selenous acid added to the culture medium is preferably 1000 µg/L, more preferably 500 µg/L, more preferably 100 µg/L, more preferably 90 µg/L, more preferably 80 µg/L, more preferably 70 µg/L, more preferably 60 µg/L, more preferably 50 µg/L, more preferably 40 µg/L, more preferably 30 µg/L, more preferably 20 µg/L, more preferably 10 µg/L, more preferably 9 µg/L, more preferably 8 µg/L, more preferably 7 µg/L, more preferably 6 µg/L, more preferably 5 µg/L, and more preferably 4 µg/L.

### [2] Differentiation-inducing factor used to induce differentiation from endodermal cells into primitive gut tube cells, and other additives

In the step (a) of the present invention, endodermal cells, which have been induced to differentiate from pluripotent stem cells, are cultured in the presence of a bone morphogenetic protein (BMP) signaling inhibitor, and retinoic acid or a retinoic acid analog under culture conditions suitable for the conditions for differentiation induction into primitive gut tube (PGT) cells to induce differentiation into primitive gut tube (PGT) cells. In the step (a), endodermal cells, which have been induced to differentiate from pluripotent stem cells, are cultured in the presence of a bone morphogenetic protein (BMP) signaling inhibitor, and retinoic acid or a retinoic acid analog to induce differentiation into primitive gut tube (PGT) cells, which makes it possible to efficiently induce differentiation from the primitive gut tube (PGT) cells into pancreatic α cells.

The culture conditions suitable for inducing differentiation into the primitive gut tube (PGT) cells are not particularly limited as long as they are culture conditions that can appropriately induce the differentiation of the endodermal cells, which have been induced to differentiate from pluripotent stem cells, into the primitive gut tube (PGT) cells.

Although the differentiation induction medium is not particularly limited as long as it is a culture medium that induces the differentiation of endodermal cells to primitive gut tube (PGT) cells, as one embodiment, the cells are cultured in the differentiation induction medium described below.

When the bone morphogenetic protein (BMP) signaling inhibitor is dorsomorphin, the lower limit of the amount of the bone morphogenetic protein (BMP) signaling inhibitor added in the culture medium used in the step (a) is preferably 0.01 µmoL/L, more preferably 0.05 µmoL/L, more preferably 0.1 µmoL/L, more preferably 0.3 µmoL/L, more preferably 0.5 µmoL/L, more preferably 0.6 µmoL/L, more preferably 0.7 µmoL/L, more preferably 0.8 µmoL/L, more preferably 0.9 µmoL/L, and more preferably 1 µmoL/L. The upper limit of the amount of dorsomorphin contained in the culture medium is preferably 20 µmoL/L, more preferably 15 µmoL/L, more preferably 10 µmoL/L, more preferably 7 µmoL/L, more preferably 5 µmoL/L, more preferably 4 µmoL/L, more preferably 3 µmoL/L, more preferably 2 µmoL/L, and more preferably 1 µmoL/L.

When the bone morphogenetic protein (BMP) signaling inhibitor is LDN193189, the lower limit of the amount of LDN193189 contained in the culture medium is preferably 0.01 µmoL/L, more preferably 0.02 µmoL/L, more preferably 0.05 µmoL/L, more preferably 0.1 µmoL/L, more preferably 0.2 µmoL/L, more preferably 0.3 µmoL/L, more preferably 0.4 µmoL/L, more preferably 0.5 µmoL/L, and more preferably 0.6 µmoL/L. The upper limit of the amount of LDN193189 contained in the culture medium is preferably 10 µmoL/L, more preferably 5 µmoL/L, more preferably 1 µmoL/L, more preferably 0.9 µmoL/L, more preferably 0.8 µmoL/L, and more preferably 0.7 moL/L.

The lower limit of the amount of retinoic acid or a retinoic acid analog (such as EC23) added to the culture medium used in the step (a) is preferably 0.01 µmol/L, more preferably 0.02 µmol/L, more preferably 0.03 µmol/L, more preferably 0.05 µmol/L, more preferably 0.1 µmol/L, more preferably 0.2 µmol/L, more preferably 0.3 µmol/L, more preferably 0.4 µmol/L, more preferably 0.5 µmol/L, more preferably 0.6 µmol/L, more preferably 0.7 µmol/L, more preferably 0.8 µmol/L, more preferably 0.9 µmol/L, and more preferably 1.0 µmol/L. The upper limit of the amount of the retinoic acid analog (such as EC23) added to the culture medium is preferably 5.0 µmol/L, more preferably 4.0 µmol/L, more preferably 3.0 µmol/L, more preferably 2.0 µmol/L, and more preferably 1.0 µmol/L.

The step (a) may be performed in the presence of a ROCK signaling inhibitor (such as Y27632).

When the step (a) is performed in the presence of the ROCK signaling inhibitor (such as Y27632), the lower limit of the amount of the ROCK signaling inhibitor (such as Y27632) contained in the culture medium is preferably 0.1 µmoL/L, more preferably 0.5 µmoL/L, more preferably 1 µmoL/L, more preferably 1.5 µmoL/L, more preferably 2.0 µmoL/L, and more preferably 2.5 µmoL/L. The upper limit of the amount of the ROCK signaling inhibitor (such as Y27632) contained in the culture medium is preferably 100 µmoL/L, more preferably 50 µmoL/L, more preferably 40 µmoL/L, more preferably 30 µmoL/L, more preferably 20 µmoL/L, more preferably 10 µmoL/L, more preferably 5 µmoL/L, more preferably 4 µmoL/L, more preferably 3 µmoL/L, and more preferably 2.5 µmoL/L.

The culture medium used in the step (a) is preferably a culture medium containing an insulin receptor signaling activator.

The lower limit of the amount of the insulin receptor signaling activator added to the culture medium is preferably 0.001 mg/L, more preferably 0.01 mg/L, more preferably 0.1 mg/L, more preferably 1 mg/L, more preferably 2 mg/L, more preferably 3 mg/L, more preferably 4 mg/L, and more preferably 5 mg/L. The upper limit of the amount of the insulin receptor signaling activator added to the culture medium is preferably 1000 mg/L, more preferably 500 mg/L, more preferably 100 mg/L, more preferably 90 mg/L, more preferably 80 mg/L, more preferably 70 mg/L, more preferably 60 mg/L, more preferably 50 mg/L, more preferably 40 mg/L, more preferably 30 mg/L, more preferably 20 mg/L, and more preferably 10 mg/L.

The culture medium used in the step (a) is preferably a culture medium containing insulin, transferrin, and selenous acid.

The insulin, transferrin, and selenous acid may be contained in the culture medium in the form of a commercially available mixture such as a B27 supplement. Additionally, ethanolamine may be contained in addition to insulin, transferrin, and selenous acid.

The lower limit of the amount of transferrin added to the culture medium is preferably 0.001 mg/L, more preferably 0.01 mg/L, more preferably 0.1 mg/L, more preferably 1 mg/L, more preferably 2 mg/L, more preferably 2.1 mg/L, more preferably 2.2 mg/L, more preferably 2.3 mg/L, more preferably 2.4 mg/L, more preferably 2.5 mg/L, more preferably 2.6 mg/L, and more preferably 2.7 mg/L. The upper limit of the amount of transferrin added to the culture medium is preferably 1000 mg/L, more preferably 500 mg/L, more preferably 100 mg/L, more preferably 90 mg/L, more preferably 80 mg/L, more preferably 70 mg/L, more preferably 60 mg/L, more preferably 50 mg/L, more preferably 40 mg/L, more preferably 30 mg/L, more preferably 20 mg/L, more preferably 10 mg/L, more preferably 9 mg/L, more preferably 8 mg/L, more preferably 7 mg/L, more preferably 6 mg/L, more preferably 5 mg/L, more preferably 4 mg/L, more preferably 3 mg/L, more preferably 2.9 mg/L, and more preferably 2.8 mg/L.

The lower limit of the amount of selenous acid added to the culture medium is preferably 0.001 µg/L, more preferably 0.01 µg/L, more preferably 0.1 µg/L, more preferably 1 µg/L, more preferably 2 µg/L, more preferably 2.1 µg/L, more preferably 2.2 µg/L, more preferably 2.3 µg/L, more preferably 2.4 µg/L, more preferably 2.5 µg/L, more preferably 2.6 µg/L, more preferably 2.7 µg/L, more preferably 2.8 µg/L, more preferably 2.9 µg/L, more preferably 3 µg/L, more preferably 3.1 µg/L, more preferably 3.2 µg/L, and more preferably 3.3 µg/L. The upper limit of the amount of selenous acid added to the culture medium is preferably 1000 µg/L, more preferably 500 µg/L, more preferably 100 µg/L, more preferably 90 µg/L, more preferably 80 µg/L, more preferably 70 µg/L, more preferably 60 µg/L, more preferably 50 µg/L, more preferably 40 µg/L, more preferably 30 µg/L, more preferably 20 µg/L, more preferably 10 µg/L, more preferably 9 µg/L, more preferably 8 µg/L, more preferably 7 µg/L, more preferably 6 µg/L, more preferably 5 µg/L, and more preferably 4 µg/L.

The culture medium used in the step (a) is preferably a culture medium containing a FGF receptor signaling activator. However, for the purpose of achieving more efficient differentiation induction, the culture in the step (a) is preferably performed in the absence of FGF2.

The lower limit of the amount of the FGF receptor signaling activator added to the culture medium is preferably 1 ng/mL, more preferably 5 ng/mL, more preferably 10 ng/mL, more preferably 20 ng/mL, more preferably 30 ng/mL, more preferably 40 ng/mL, and more preferably 50 ng/mL. The upper limit of the amount of the FGF receptor signaling activator added to the culture medium is preferably 500 ng/mL, more preferably 400 ng/mL, more preferably 300 ng/mL, more preferably 200 ng/mL, more preferably 100 ng/mL, more preferably 90 ng/mL, more preferably 80 ng/mL, more preferably 70 ng/mL, more preferably 60 ng/mL, and more preferably 50 ng/mL.

The culture medium used in the step (a) is preferably a culture medium containing a B27 (registered trademark) supplement and/or FGF7.

The lower limit of the amount of the B27 (registered trademark) supplement added to the culture medium is preferably 0.01 %, more preferably 0.1 %, more preferably 0.2%, more preferably 0.3%, more preferably 0.4%, more preferably 0.5%, more preferably 0.6%, more preferably 0.7%, more preferably 0.8%, and more preferably 0.9%. The upper limit of the amount of the B27 (registered trademark) supplement added to the culture medium is preferably 10%, more preferably 9%, more preferably 8%, more preferably 7%, more preferably 6%, more preferably 5%, more preferably 4%, more preferably 3%, more preferably 2%, and more preferably 1%.

The lower limit of the amount of FGF7 added to the culture medium is preferably 1 ng/mL, more preferably 5 ng/mL, more preferably 10 ng/mL, more preferably 20 ng/mL, more preferably 30 ng/mL, more preferably 40 ng/mL, and more preferably 50 ng/mL. The upper limit of the amount of FGF7 added to the culture medium is preferably 500 ng/mL, more preferably 400 ng/mL, more preferably 300 ng/mL, more preferably 200 ng/mL, more preferably 100 ng/mL, more preferably 90 ng/mL, more preferably 80 ng/mL, more preferably 70 ng/mL, more preferably 60 ng/mL, and more preferably 50 ng/mL.

The culture in the step (a) is a culture in the absence of a hedgehog (HH) signaling inhibitor, and is more preferably a culture in the absence of the TGFβ signaling inhibitor.

As the culture medium, in accordance with the types of cells that are used, an MEM medium, a BME medium, a DMEM medium, a DMEM/F12 medium, an αMEM medium, an IMDM medium, an ES medium, a DM-160 medium, a Fisher medium, an F12 medium, a WE medium, an RPMI1640 medium, an Essential 6^{™} medium (Thermo Fisher Scientific), or mixed media thereof, or the like may be used.

The culture medium may further contain bovine serum albumin (BSA) or human serum albumin (HSA). Preferably, the BSA or HSA contains 2 mg/g or less of lipids and 0.2 mg/g or less of free fatty acids.

The lower limit of the amount of BSA added to the culture medium is preferably 0.01% (% by weight), more preferably 0.05%, more preferably 0.10%, more preferably 0.15%, more preferably 0.20%, and more preferably 0.25%. The upper limit of the amount of BSA added to the culture medium is preferably 1.00%, more preferably 0.90%, more preferably 0.80%, more preferably 0.70%, more preferably 0.60%, more preferably 0.50%, more preferably 0.40%, more preferably 0.30%, and more preferably 0.25%.

The culture medium may further contain sodium pyruvate.

The lower limit of the amount of sodium pyruvate added to the culture medium is preferably 0.01 mmol/L, more preferably 0.05 mmol/L, more preferably 0.1 mmol/L, more preferably 0.2 mmol/L, more preferably 0.5 mmol/L, more preferably 0.6 mmol/L, more preferably 0.7 mmol/L, more preferably 0.8 mmol/L, more preferably 0.9 mmol/L, and more preferably 1 mmol/L. The upper limit of the amount of sodium pyruvate added to the culture medium is preferably 20 mmol/L, more preferably 15 mmol/L, more preferably 10 mmol/L, more preferably 5 mmol/L, more preferably 4 mmol/L, more preferably 3 mmol/L, more preferably 2 mmol/L, and more preferably 1 mmol/L.

The culture medium may further contain NEAA (for example, 1 × non-essential amino acids (NEAA, Wako) or the like).

The lower limit of the amount of NEAA contained in the culture medium is preferably 0.05 × NEAA, more preferably 0.1 × NEAA, more preferably 0.5 × NEAA, more preferably 0.6 × NEAA, more preferably 0.7 × NEAA, more preferably 0.8 × NEAA, more preferably 0.9 × NEAA, and more preferably 1 × NEAA. The upper limit of the amount of NEAA contained in the culture medium is preferably 20 × NEAA, more preferably 15 × NEAA, more preferably 10 × NEAA, more preferably 5 × NEAA, more preferably 4 × NEAA, more preferably 3 × NEAA, more preferably 2 × NEAA, and more preferably 1 × NEAA.

The culture medium may further contain a growth factor stabilizer (such as heparin).

The lower limit of the amount of the growth factor stabilizer (such as heparin) added to the culture medium is preferably 0.2 µg/mL, more preferably 0.5 µg/mL, more preferably 1 µg/mL, more preferably 2 µg/mL, more preferably 3 µg/mL, more preferably 4 µg/mL, and more preferably 5 µg/mL. The upper limit of the amount of the growth factor stabilizer (such as heparin) added to the culture medium is preferably 100 µg/mL, more preferably 90 µg/mL, more preferably 80 µg/mL, more preferably 70 µg/mL, more preferably 60 µg/mL, more preferably 50 µg/mL, more preferably 40 µg/mL, more preferably 30 µg/mL, more preferably 20 µg/mL, more preferably 10 µg/mL, more preferably 9 µg/mL, more preferably 8 µg/mL, more preferably 7 µg/mL, more preferably 6 µg/mL, and more preferably 5 µg/mL.

The culture medium may further contain antibiotics such as penicillin and streptomycin.

The culture temperature is not particularly limited as long as it is a culture temperature suitable for culturing the pluripotent stem cells that are used. Generally, the temperature is 30°C to 40°C, and is preferably about 37°C.

The cells are preferably cultured by using a CO₂ incubator or the like in an atmosphere with a CO₂ concentration of about 1% to 10%, preferably 5%.

The culture may be implemented while stirring. Although the stirring speed is not particularly limited, the upper limit is preferably 200 rpm, more preferably 150 rpm, even more preferably 120 rpm, more preferably 110 rpm, more preferably 100 rpm, more preferably 90 rpm, more preferably 80 rpm, more preferably 70 rpm, particularly preferably 60 rpm, and most preferably 55 rpm. The lower limit is preferably 1 rpm, more preferably 10 rpm, even more preferably 20 rpm, more preferably 30 rpm, more preferably 40 rpm, particularly preferably 50 rpm, and most preferably 55 rpm.

### [3] Differentiation-inducing factor used to induce differentiation from primitive gut tube cells into pancreatic endocrine precursor cells, other additives, and culture conditions

In the step (b) of the present invention, primitive gut tube (PGT) cells, which have been induced to differentiate from endodermal cells, are induced to differentiate into pancreatic endocrine precursor (EP) cells under culture conditions suitable for inducing differentiation into pancreatic endocrine precursor (EP) cells.

The culture conditions suitable for inducing differentiation into the pancreatic endocrine precursor (EP) cells are not particularly limited as long as they are culture conditions that can appropriately induce the differentiation of the primitive gut tube (PGT) cells, which have been induced to differentiate from endodermal cells, into the pancreatic endocrine precursor (EP) cells.

Although the differentiation induction medium is not particularly limited as long as it is a culture medium that induces the differentiation of primitive gut tube (PGT) cells into pancreatic endocrine precursor (EP) cells, as one embodiment, the cells are cultured in the differentiation induction medium described below.

The step (b) in the present invention is performed in the absence of ascorbic acid from the viewpoint of efficiently inducing differentiation from primitive gut tube (PGT) cells into pancreatic α cells.

The step (b) in the present invention includes a step of inducing differentiation from primitive gut tube (PGT) cells which have been induced to differentiate from endodermal cells into posterior foregut (PFG) cells, a step of inducing differentiation from the posterior foregut (PFG) cells into pancreatic progenitor (PP) cells, and a step of inducing differentiation from the pancreatic progenitor (PP) cells into pancreatic endocrine precursor (EP) cells.

The step (b) in the present invention may include the following steps:
a step (b1) of culturing the primitive gut tube (PGT) cells in the presence of a protein kinase C (PKC) activator to induce differentiation into posterior foregut (PFG) cells,
a step (b2) of culturing the posterior foregut (PFG) cells in the presence of retinoic acid or an analog thereof to induce differentiation into pancreatic progenitor (PP) cells, and
a step (b3) of culturing the pancreatic progenitor (PP) cells in the presence of a Notch signaling inhibitor and a ROCK signaling inhibitor to induce differentiation into pancreatic endocrine precursor (EP) cells.

### [3-1] Differentiation-inducing factor used to induce differentiation from primitive gut tube cells into posterior foregut cells, and other additives

In the step (b1), primitive gut tube (PGT) cells, which have been induced to differentiate from endodermal cells, are cultured in the presence of a protein kinase (PKC) activator under culture conditions suitable for inducing differentiation into posterior foregut (PFG) cells, thereby inducing differentiation into posterior foregut (PFG) cells.

The culture conditions suitable for inducing differentiation into posterior foregut (PFG) cells are not particularly limited as long as they are culture conditions that can appropriately induce the differentiation of primitive gut tube (PGT) cells, which have been induced to differentiate from endodermal cells, into posterior foregut (PFG) cells.

Although the differentiation induction medium is not particularly limited as long as it is a culture medium that induces the differentiation of primitive gut tube (PGT) cells into posterior foregut (PFG) cells, as one embodiment, the cells are cultured in the differentiation induction medium described below.

The lower limit of the amount of the PKC activator contained in the culture medium used in the aforementioned step (b1) is preferably 0.01 µmoL/L, more preferably 0.02 µmoL/L, more preferably 0.05 µmoL/L, more preferably 0.1 µmoL/L, more preferably 0.2 µmoL/L, and more preferably 0.3 µmoL/L. The upper limit of the amount of the PKC activator contained in the culture medium is preferably 10 µmoL/L, more preferably 5 µmoL/L, more preferably 1 µmoL/L, more preferably 0.8 µmoL/L, more preferably 0.5 µmoL/L, more preferably 0.4 moL/L, and more preferably 0.3 µmoL/L.

The culture medium used in the aforementioned step (b1) may contain a hedgehog (HH) signaling inhibitor (such as SANT1).

The lower limit of the amount of the HH signaling inhibitor (such as SANT1) added to the culture medium is preferably 0.01 µmol/L, more preferably 0.02 µmol/L, more preferably 0.03 µmol/L, more preferably 0.05 µmol/L, more preferably 0.10 µmol/L, more preferably 0.15 µmol/L, more preferably 0.20 µmol/L, and more preferably 0.25 µmol/L. The upper limit of the amount of the HH signaling inhibitor (such as SANT1) added to the culture medium is preferably 5.0 µmol/L, more preferably 4.0 µmol/L, more preferably 3.0 µmol/L, more preferably 2.0 µmol/L, more preferably 1.0 µmol/L, more preferably 0.80 µmol/L, more preferably 0.70 µmol/L, more preferably 0.60 µmol/L, more preferably 0.50 µmol/L, more preferably 0.40 µmol/L, more preferably 0.30 µmol/L, and more preferably 0.25 µmol/L.

The culture medium used in the step (b1) may contain a bone morphogenetic protein (BMP) signaling inhibitor.

In the case in which the bone morphogenetic protein (BMP) signaling inhibitor is LDN193189, the lower limit of the amount of LDN193189 contained in the culture medium is preferably 0.01 µmoL/L, more preferably 0.02 µmoL/L, more preferably 0.05 µmoL/L, more preferably 0.1 µmoL/L, and more preferably 0.2 µmoL/L. The upper limit of the amount of LDN193189 contained in the culture medium is preferably 10 µmoL/L, more preferably 5 µmoL/L, more preferably 1 µmoL/L, more preferably 0.8 µmoL/L, more preferably 0.5 µmoL/L, more preferably 0.4 µmoL/L, more preferably 0.3 µmoL/L, and more preferably 0.2 µmoL/L.

The culture medium used in the step (b1) may contain an EGF receptor signaling activator.

The lower limit of the amount of the EGF receptor signaling activator added to the culture medium is preferably 1 ng/mL, more preferably 5 ng/mL, more preferably 10 ng/mL, more preferably 15 ng/mL, more preferably 20 ng/mL, and more preferably 25 ng/mL. The upper limit of the amount of the EGF receptor signaling activator added to the culture medium is preferably 500 ng/mL, more preferably 400 ng/mL, more preferably 300 ng/mL, more preferably 200 ng/mL, more preferably 100 ng/mL, more preferably 50 ng/mL, more preferably 40 ng/mL, more preferably 30 ng/mL, and more preferably 25 ng/mL.

The culture medium used in the step (b1) may contain a retinoic acid analog (such as EC23). The amount of the retinoic acid analog added to the culture medium is as described in [2] above.

The culture medium used in the step (b1) may contain NEAA (such as, for example, 1 × non-essential amino acids (NEAA, Wako)). The amount of the NEAA added to the culture medium is as described in [2] above.

The culture medium in the step (b1) may contain an FGF receptor signaling activator. Particularly preferably, it contains FGF7. The amount of the FGF receptor signaling activator added to the culture medium is as described in [2] above.

### [3-2] Differentiation-inducing factor used to induce differentiation from posterior foregut (PFG) cells into pancreatic progenitor (PP) cells, and other additives

In the step (b2), posterior foregut (PFG) cells are cultured in the presence of retinoic acid or an analog thereof under culture conditions suitable for inducing differentiation into pancreatic progenitor (PP) cells, thereby inducing differentiation into pancreatic progenitor (PP) cells.

The culture conditions suitable for inducing differentiation to pancreatic progenitor (PP) cells are not particularly limited as long as they are culture conditions that can appropriately induce the differentiation of posterior foregut (PFG) cells to pancreatic progenitor (PP) cells.

Although the differentiation induction medium is not particularly limited as long as it is a culture medium that induces the differentiation of posterior foregut (PFG) cells to pancreatic progenitor (PP) cells, as one embodiment, the cells are cultured in the differentiation induction medium described below.

The amount of retinoic acid or a retinoic acid analog (such as EC23) added to the culture medium used in the step (b2) is as described in [2] above.

The culture medium used in the step (b2) may contain a hedgehog (HH) signaling inhibitor (such as SANT1). The amount of the hedgehog (HH) signaling inhibitor (such as SANT1) added to the culture medium is as described in [3-1] above.

Additionally, the culture medium used in the step (b2) may contain NEAA (such as, for example, 1 × non-essential amino acids (NEAA, Wako)). The amount of the NEAA added to the culture medium is as described in [2] above.

The culture medium in the step (b2) may contain a bone morphogenetic protein (BMP) signaling inhibitor. The amount of the bone morphogenetic protein (BMP) signaling inhibitor added to the culture medium is as described in [3-1] above.

The culture medium in the step (b2) may contain an FGF receptor signaling activator. Particularly preferably, it is FGF10. The amount of the FGF receptor signaling activator added to the culture medium is as described in [2] above.

The culture medium in the step (b2) may contain a protein kinase C (PKC) activator. The amount of the protein kinase C (PKC) activator added to the culture medium is as described in [3-1] above.

The culture medium in the step (b2) may contain an EGF receptor signaling activator. The amount of the EGF receptor signaling activator added to the culture medium is as described in [3-1] above.

The culture medium in the step (b2) may contain a TGF-β receptor signaling inhibitor (such as RepSOX).

The lower limit of the amount of the TGF-β receptor signaling inhibitor (such as RepSOX) contained in the culture medium is preferably 0.01 µmoL/L, more preferably 0.05 µmoL/L, more preferably 0.1 µmoL/L, more preferably 0.3 µmoL/L, more preferably 0.5 µmoL/L, more preferably 0.6 µmoL/L, more preferably 0.7 µmoL/L, more preferably 0.8 µmoL/L, more preferably 0.9 µmoL/L, and more preferably 1.0 µmoL/L. The upper limit of the amount of the TGF-β receptor signaling inhibitor (such as RepSOX) contained in the culture medium is preferably 10 µmoL/L, more preferably 8 µmoL/L, more preferably 5 µmoL/L, more preferably 4 µmoL/L, more preferably 3 µmoL/L, more preferably 2 µmoL/L, more preferably 1.5 µmoL/L, more preferably 1.2 µmoL/L, and more preferably 1.0 µmoL/L.

The culture medium in the step (b2) may contain ZnSO₄.

The lower limit of the amount of ZnSO₄ contained in the culture medium is preferably 0.1 µmoL/L, more preferably 0.5 µmoL/L, more preferably 1 µmoL/L, more preferably 3 µmoL/L, and more preferably 5 µmoL/L. The upper limit of the amount of ZnSO₄ contained in the culture medium is preferably 100 µmoL/L, more preferably 80 µmoL/L, more preferably 50 µmoL/L, more preferably 40 µmoL/L, more preferably 30 µmoL/L, more preferably 20 µmoL/L, more preferably 15 µmoL/L more preferably 12 µmoL/L, more preferably 10 µmoL/L, and more preferably 5 µmoL/L.

### [3-3] Differentiation-inducing factor used to induce differentiation from pancreatic progenitor (PP) cells into pancreatic endocrine precursor (EP) cells, and other additives

In the step (b3) in the present invention, pancreatic progenitor (PP) cells are cultured in the presence of a Notch signaling inhibitor and a ROCK signaling inhibitor under culture conditions suitable for inducing differentiation into pancreatic endocrine precursor (EP) cells, thereby inducing differentiation into pancreatic endocrine precursor (EP) cells.

The culture conditions suitable for inducing differentiation into endocrine precursor (EP) cells are not particularly limited as long as they are culture conditions that can appropriately induce the differentiation of posterior foregut (PP) cells into pancreatic endocrine precursor (EP) cells.

Although the differentiation induction medium is not particularly limited as long as it is a culture medium that induces the differentiation of posterior foregut (PP) cells to pancreatic endocrine precursor (EP) cells, as one embodiment, the cells are cultured in the differentiation induction medium described below.

The lower limit of the amount of the Notch signaling inhibitor (such as DBZ) in the culture medium in the aforementioned step (b3) is preferably 0.01 µmol/L, more preferably 0.02 µmol/L, more preferably 0.03 µmol/L, more preferably 0.05 µmol/L, more preferably 0.1 µmol/L, more preferably 0.2 µmol/L, more preferably 0.3 µmol/L, more preferably 0.4 µmol/L, and more preferably 0.5 µmol/L. The upper limit of the amount of the Notch signaling inhibitor (such as DBZ) added to the culture medium is preferably 5.0 µmol/L, more preferably 4.0 µmol/L, more preferably 3.0 µmol/L, more preferably 2.0 µmol/L, more preferably 1.0 µmol/L, more preferably 0.9 µmol/L, more preferably 0.8 µmol/L, more preferably 0.7 µmol/L, more preferably 0.6 µmol/L, and more preferably 0.5 µmol/L.

The culture medium used in the step (b3) may contain a hedgehog (HH) signaling inhibitor (such as SANT1). The amount of the hedgehog (HH) signaling inhibitor (such as SANT1) added to the culture medium is as described in [3-1] above.

The culture medium used in the step (b3) may contain a retinoic acid analog (such as EC23). The amount of the retinoic acid analog added to the culture medium is as described in [2] above.

The culture medium used in the step (b3) may contain L-glutamine.

The lower limit of the amount of L-glutamine contained in the culture medium is preferably 0.01 mmol/L, more preferably 0.05 mmol/L, more preferably 0.1 mmol/L, more preferably 0.5 mmol/L, more preferably 0.7 mmol/L, more preferably 1.0 mmol/L, more preferably 1.2 mmol/L, more preferably 1.5 mmol/L, and more preferably 2.0 mmol/L. The upper limit of the amount of L-glutamine contained in the culture medium is preferably 100 mmol/L, more preferably 50 mmol/L, more preferably 40 mmol/L, more preferably 30 mmol/L, more preferably 20 mmol/L, more preferably 10 mmol/L, more preferably 9 mmol/L, more preferably 8 mmol/L, more preferably 7 mmol/L, more preferably 6 mmol/L, more preferably 5 mmol/L, more preferably 4 mmol/L, more preferably 3 mmol/L, and more preferably 2 mmol/L.

The culture medium in the step (b3) may contain a bone morphogenetic protein (BMP) signaling inhibitor. The amount of the bone morphogenetic protein (BMP) signaling inhibitor added to the culture medium is as described in [3-1] above.

The culture medium in the step (b3) may contain a TGF-β receptor signaling inhibitor. The amount of the TGF-β receptor signaling inhibitor added to the culture medium is as described in [3-2] above.

The culture medium in the step (b3) may contain ZnSO₄. The amount of the ZnSO₄ added to the culture medium is as described in [3-2] above.

The culture medium in the step (b3) may contain a growth factor stabilizer (such as heparin). The amount of the growth factor stabilizer (such as heparin) added to the culture medium is as described in [2] above.

The culture medium in the step (b3) may contain nicotinamide.

The lower limit of the amount of nicotinamide contained in the culture medium is preferably 0.1 mmoL/L, more preferably 0.5 mmoL/L, more preferably 1 mmoL/L, more preferably 3 mmoL/L, and more preferably 5 mmoL/L. The upper limit of the amount of nicotinamide in the culture medium is preferably 100 mmoL/L, more preferably 80 mmoL/L, more preferably 50 mmoL/L, more preferably 40 mmoL/L, more preferably 30 mmoL/L, more preferably 20 mmoL/L, more preferably 15 mmoL/L, more preferably 12 mmoL/L, more preferably 10 mmoL/L, and more preferably 5 mmoL/L.

The culture medium in the step (b3) may contain an EGF receptor signaling activator (such as EGF). The amount of the EGF receptor signaling activator (such as EGF) added to the culture medium is as described in [3-1] above.

The culture medium in the step (b3) may contain a ROCK signaling inhibitor (such as Y27632).

When the step (b3) is performed in the presence of the ROCK signaling inhibitor (such as Y27632), the lower limit of the amount of the ROCK signaling inhibitor (such as Y27632) contained in the culture medium is preferably 0.1 µmoL/L, more preferably 0.5 µmoL/L, more preferably 1 µmoL/L, more preferably 3 µmoL/L, more preferably 5 µmoL/L, more preferably 6 µmoL/L, more preferably 7 µmoL/L, more preferably 8 µmoL/L, more preferably 9 µmoL/L, and more preferably 10 µmoL/L. The upper limit of the amount of the ROCK signaling inhibitor (such as Y27632) contained in the culture medium is preferably 100 µmoL/L, more preferably 80 µmoL/L, more preferably 50 µmoL/L, more preferably 40 µmoL/L, more preferably 30 µmoL/L, more preferably 20 µmoL/L, and more preferably 10 µmoL/L.

### [4] Differentiation-inducing factor used to induce differentiation from pancreatic endocrine precursor (EP) cells into pancreatic α cells, other additives, and culture conditions

In the step (c) of the present invention, pancreatic endocrine precursor (EP) cells are cultured under conditions suitable for inducing differentiation into pancreatic α cells, thereby producing pancreatic α cells.

The culture conditions suitable for inducing differentiation into pancreatic α cells are not particularly limited as long as they are culture conditions that can appropriately induce the differentiation of pancreatic endocrine precursor (EP) cells into pancreatic α cells.

Although the differentiation induction medium is not particularly limited as long as it is a culture medium that induces the differentiation of pancreatic endocrine precursor (EP) cells into pancreatic α cells, as one embodiment, the cells are cultured in the differentiation induction medium described below.

The step (c) in the present invention is performed in the absence of ascorbic acid from the viewpoint of efficiently inducing differentiation from pancreatic endocrine precursor (EP) cells into pancreatic α cells.

The step (c) in the present invention is preferably performed under oxygen supply conditions. By performing the step (c) under the oxygen supply conditions, pancreatic endocrine precursor (EP) cells can be efficiently induced to differentiate into pancreatic α cells.

The oxygen supply condition is preferably a condition in which a dissolved oxygen concentration in a culture solution is controlled to be 20% to 50% when a saturated dissolved oxygen concentration in the culture solution at 37°C at 1 atm is 100%.

The dissolved oxygen concentration in the culture solution can be measured by a dissolved oxygen meter such as a fluorescence type oxygen sensor or a galvanic cell type oxygen sensor, for example.

The oxygen supply is not particularly limited as long as it is a method that can supply oxygen to the culture medium. Examples thereof include surface ventilation in which air or oxygen is ventilated on the upper surface of culture solution, a sparging method in which air or oxygen is supplied to the culture solution using a sparger or the like, and a method in which oxygen is supplied to the culture solution using microbubbles or nanobubbles, among which surface ventilation is preferable. Furthermore, these oxygen supply methods can be combined with the case of performing the step (c) under stirring culture. The stirring conditions in the stirring culture are not particularly limited as long as they are in the range in which oxygen is supplied to the culture solution. The upper limit is preferably 200 rpm, more preferably 150 rpm, even more preferably 120 rpm, more preferably 100 rpm, more preferably 90 rpm, more preferably 80 rpm, further more preferably 70 rpm, particularly preferably 60 rpm, and most preferably 55 rpm. The lower limit is preferably 1 rpm, more preferably 10 rpm, even more preferably 20 rpm, more preferably 30 rpm, more preferably 40 rpm, particularly preferably 50 rpm, and most preferably 55 rpm.

In the step (c) in the present invention, the pancreatic endocrine precursor (EP) cells may be cultured in the presence of an insulin receptor signaling activator, transferrin, and selenous acid.

The amounts of the insulin receptor signaling activator, transferrin, and selenous acid added to the culture medium are as described in <Culture medium> above.

The culture medium in the step (c) may contain a TGF-β receptor signaling inhibitor. The amount of the TGF-β receptor signaling inhibitor added to the culture medium is as described in [3-2] above.

The culture medium in the step (c) may contain ZnSO₄. The amount of the ZnSO₄ added to the culture medium is as described in [3-2] above.

The culture medium in the step (c) may contain a GLP-1 (glucagon-like peptide-1) receptor signaling activator (such as Exendin 4).

The lower limit of the amount of the GLP-1 (glucagon-like peptide-1) receptor signaling activator (such as Exendin 4) added to the culture medium is preferably 1 ng/mL, more preferably 5 ng/mL, more preferably 10 ng/mL, more preferably 20 ng/mL, more preferably 30 ng/mL, more preferably 40 ng/mL, and more preferably 50 ng/mL. The upper limit of the amount of the GLP-1 receptor signaling activator (such as Exendin 4) added to the culture medium is preferably 500 ng/mL, more preferably 400 ng/mL, more preferably 300 ng/mL, more preferably 200 ng/mL, more preferably 100 ng/mL, more preferably 90 ng/mL, more preferably 80 ng/mL, more preferably 70 ng/mL, more preferably 60 ng/mL, and more preferably 50 ng/mL.

The culture medium in the step (c) may contain a growth factor stabilizer (such as heparin). The amount of the growth factor stabilizer (such as heparin) added to the culture medium is as described in [2] above.

The culture medium in the step (c) may contain nicotinamide. The amount of nicotinamide added to the culture medium is as described in [3-3] above.

The culture medium in the step (c) may contain a TGFβ superfamily signaling activator (such as BMP4).

The lower limit of the amount of the TGFβ superfamily signaling activator (such as BMP4) added to the culture medium is preferably 0.2 ng/mL, more preferably 0.5 ng/mL, more preferably 1 ng/mL, more preferably 3 ng/mL, more preferably 5 ng/mL, more preferably 6 ng/mL, more preferably 7 ng/mL, more preferably 8 ng/mL, more preferably 9 ng/mL, and more preferably 10 ng/mL. The upper limit of the amount of the TGFβ superfamily signaling activator (such as BMP4) added to the culture medium is preferably 100 ng/mL, more preferably 90 ng/mL, more preferably 80 ng/mL, more preferably 70 ng/mL, more preferably 60 ng/mL, more preferably 50 ng/mL, more preferably 40 ng/mL, more preferably 30 ng/mL, more preferably 20 ng/mL, and more preferably 10 ng/mL.

The culture medium in the step (c) may contain a hepatocyte growth factor (HGF).

The lower limit of the amount of the hepatocyte growth factor (HGF) added to the culture medium is preferably 1 ng/mL, more preferably 5 ng/mL, more preferably 10 ng/mL, more preferably 20 ng/mL, more preferably 30 ng/mL, more preferably 40 ng/mL, and more preferably 50 ng/mL. The upper limit of the amount of the hepatocyte growth factor (HGF) added to the culture medium is preferably 500 ng/mL, more preferably 400 ng/mL, more preferably 300 ng/mL, more preferably 200 ng/mL, more preferably 100 ng/mL, more preferably 90 ng/mL, more preferably 80 ng/mL, more preferably 70 ng/mL, more preferably 60 ng/mL, and more preferably 50 ng/mL.

The culture medium in the step (c) may contain an insulin-like growth factor (such as IGF1).

The lower limit of the amount of the insulin-like growth factor (such as IGF1) added to the culture medium is preferably 1 ng/mL, more preferably 5 ng/mL, more preferably 10 ng/mL, more preferably 20 ng/mL, more preferably 30 ng/mL, more preferably 40 ng/mL, and more preferably 50 ng/mL. The upper limit of the amount of the insulin-like growth factor (such as IGF1) added to the culture medium is preferably 500 ng/mL, more preferably 400 ng/mL, more preferably 300 ng/mL, more preferably 200 ng/mL, more preferably 100 ng/mL, more preferably 90 ng/mL, more preferably 80 ng/mL, more preferably 70 ng/mL, more preferably 60 ng/mL, and more preferably 50 ng/mL.

The culture medium in the step (c) may contain an adenylate cyclase activator (such as forskolin).

The lower limit of the amount of the adenylate cyclase activator (such as forskolin) contained in the culture medium is preferably 0.1 µmoL/L, more preferably 0.3 µmoL/L, more preferably 0.5 µmoL/L, more preferably 0.7 µmol/L, more preferably 1 µmoL/L, more preferably 2 µmoL/L, more preferably 3 µmoL/L, more preferably 4 µmoL/L, and more preferably 5 µmoL/L. The upper limit of the amount of the adenylate cyclase activator (such as forskolin) contained in the culture medium is preferably 100 µmoL/L, more preferably 80 µmoL/L, more preferably 50 µmoL/L, more preferably 40 µmoL/L, more preferably 30 µmoL/L, more preferably 20 µmoL/L, more preferably 15 µmoL/L, more preferably 10 µmoL/L, more preferably 7 µmoL/L, and more preferably 5 µmoL/L.

### [5] Maintenance culture of pluripotent stem cells

In the method for inducing differentiation of the present invention, endodermal cells induced to differentiate from pluripotent stem cells are used.

The undifferentiated state of the pluripotent stem cells before being induced to differentiate into endodermal cells is preferably maintained by using an undifferentiated-state maintenance medium. A culture in which the undifferentiated state of pluripotent stem cells is maintained by using the undifferentiated-state maintenance medium is also called a maintenance culture of pluripotent stem cells.

The undifferentiated-state maintenance medium is not particularly limited as long as it is a culture medium that allows the undifferentiated state of pluripotent stem cells to be maintained. Examples include a culture medium containing a leukemia inhibitory factor that is known to have the property of maintaining the undifferentiated state of mouse embryonic stem cells and mouse induced pluripotent stem cells, a culture medium containing a basic FGF (fibroblast growth factor) that is known to have the property of maintaining the undifferentiated state of human iPS cells, and the like. For example, it is possible to use a human iPS cell medium (DMEM/Ham's F12 (Wako) containing 20% KnockOut serum replacement (KSR; Gibco), 1 × non-essential amino acids (NEAA; Wako), 55 µmol/L 2-mercaptoethanol (2-ME; Gibco), 7.5 ng/mL recombinant human fibroblast growth factor 2 (FGF 2; PeproTech) and 0.5 × penicillin and streptomycin (PS; Wako)), or an Essentail 8 medium (Thermo Fisher Scientific), STEMPRO (registered trademark) hESC SFM (Life Technologies Japan Ltd.), mTeSR1 (Veritas Corporation), TeSR2 (Veritas Corporation), StemFit (registered trademark), or the like, but there is no particular limitation.

The pluripotent stem cells may be maintenance-cultured on suitable feeder cells (for example, SL10 feeder cells, SNL feeder cells, or the like) using an undifferentiated-state maintenance medium as mentioned above. Additionally, the pluripotent stem cells may be maintenance-cultured using the above-mentioned undifferentiated-state maintenance medium on cell culture dishes coated with a cell adhesion protein or an extracellular matrix such as vitronectin, fibronectin, laminin, collagen or matrigel.

The culture temperature is not particularly limited as long as it is a culture temperature suitable for culturing the pluripotent stem cells that are used. Generally, the temperature is 30°C to 40°C, and is preferably about 37°C.

The cells are preferably cultured by using a CO₂ incubator or the like in an atmosphere with a CO₂ concentration of about 1% to 10%, preferably 5%.

The maintenance culture of the pluripotent stem cells may be performed for a desired period of time by subculturing the cells, and it is preferable to form aggregates and induce differentiation by using the pluripotent stem cells, for example, 1 to 100 passages, preferably 10 to 50 passages, more preferably 25 to 40 passages after the maintenance culture.

### [6] Formation of aggregates by suspension culturing pluripotent stem cells

As one of the embodiments for forming an aggregate of pluripotent stem cells, cells that have been maintenance-cultured in the undifferentiated state may be detached from feeder cells by using Accutase (Innovative Cell Technologies, Inc.), Accumax (Innovative Cell Technologies, Inc.) or the like, and the feeder cells are removed by rinsing three or four times with a human iPS cell culture medium. Next, the cells are broken up by pipetting into smaller cell clumps or single cells. Then, the cells are suspended in a culture medium, and thereafter suspension cultured while stirring or rotating until the pluripotent stem cells in the suspension form aggregates.

The suspension culture may be a static culture using the viscosity of the culture medium or the like, a microwell having recesses and protrusions or the like, or may involve culturing the cells under conditions in which a liquid culture medium flows with the use of a spinner or the like. Preferably, the suspension culture involves culturing the cells under conditions in which a liquid culture medium flows. Culturing the cells under conditions in which a liquid culture medium flows preferably involves culturing the cells under conditions in which the liquid culture medium flows so as to promote cell aggregation. Examples of culturing cells under conditions in which the liquid culture medium flows so as to promote cell aggregation include culturing the cells under conditions in which the liquid culture medium flows so that stresses (centrifugal force and centripetal force) due to flow such as rotational flow and rocking flow cause the cells to gather at one point, and culturing the cells under conditions such that the liquid culture medium flows with linear reciprocating motion. The cells are particularly preferably cultured by using rotational flow and/or rocking flow. Furthermore, the cells may be suspension cultured by being adhered to a microcarrier or the like in advance or may be suspension cultured in the form of cell clumps composed only of cells, or a polymer such as collagen may be intermixed into the cell clumps. Thus, the form of the culture is not particularly limited.

The culture vessel used for the suspension culture is preferably a vessel with low cell adhesion to the inner surfaces of the vessel. Such vessels with low cell adhesion to the inner surfaces of the vessel include, for example, plates that have been surface-treated for hydrophilization with a biocompatible material. For example, Nunclon^{™} Sphera (Thermo Fisher Scientific) may be used, but there is no limitation thereon. Additionally, the shape of the culture vessel is not particularly limited, and examples include culture vessels in the shape of a dish, a flask, a well, a bag, a spinner flask, or the like.

The period of time over which the aggregates are formed is not particularly limited as long as the period exceeds 6 hours. Specifically, the aggregates are preferably formed over a period of 1 day, 2 days, 3 days, 4 days, 5 days, 6 days, 7 days, or 2 weeks, 3 weeks, 4 weeks, 5 weeks, 6 weeks, 7 weeks, or 8 weeks.

The suspension culture medium is not particularly limited as long as it contains components that allow pluripotent stem cells to proliferate. An mTeSR1 (Veritas Corporation) culture medium containing 1 to 100 µM of Y-27632 (Cayman) or Essential 8^{™} containing 1 to 100 µM of Y-27632 (Cayman) and 1 to 100 mg/mL of BSA, or the like may be used.

The conditions for stirring or rotating the suspension culture are not particularly limited as long as they are within a range allowing pluripotent stem cells to form aggregates in the suspension. The upper limit is preferably 200 rpm, more preferably 150 rpm, even more preferably 120 rpm, more preferably 100 rpm, more preferably 90 rpm, more preferably 80 rpm, more preferably 70 rpm, more preferably 60 rpm, particularly preferably 50 rpm, and most preferably 45 rpm. The lower limit is preferably 1 rpm, more preferably 10 rpm, even more preferably 20 rpm, more preferably 30 rpm, more preferably 40 rpm, and particularly preferably 45 rpm. The rotation width in the case of a rotation culture is not particularly limited, and the lower limit may, for example, be 1 mm, preferably 10 mm, more preferably 20 mm, and most preferably 25 mm. The upper limit of the rotation width may, for example, be 200 mm, preferably 100 mm, preferably 50 mm, more preferably 30 mm, and most preferably 25 mm. The rotation radius in the case of a rotation culture is also not particularly limited, and is preferably set so that the rotation width is within the aforementioned range. The lower limit of the rotation radius may, for example, be 5 mm, preferably 10 mm, and the upper limit may, for example, be 100 mm, preferably 50 mm. The rotation culture conditions are preferably set to be in these ranges because these conditions make it easier for cell aggregates with appropriate dimensions to be produced.

Additionally, the suspension culture may be based on a rocking culture, in which a liquid culture medium is made to flow by rocking agitation. A rocking culture is implemented by rocking a culture vessel containing the liquid culture medium and cells in a plane substantially perpendicular to the horizontal plane. The rocking rate is not particularly limited, and the rocking can be performed with a frequency, for example, of 2 to 50 times, preferably of 4 to 25 times (one complete cycle being regarded as one time) per minute. The rocking angle is not particularly limited, and may, for example, be 0.1° to 20°, and more preferably 2° to 10°. The conditions of the rocking culture are preferably within these ranges because such conditions allow cell clumps with appropriate dimensions to be produced.

Furthermore, the cells may be cultured by agitation by means of motions combining rotation and rocking as described above.

A suspension culture using a spinner flask-shaped culture vessel is a culture that involves agitating a liquid culture medium by using a stirring blade in the culture vessel. The rotation speed and the amount of the culture medium are not particularly limited. If a commercially available spinner flask-shaped culture vessel is used, then the manufacturer-recommended amount of the culture solution may be suitably used. For example, a spinner flask from ABLE Corporation or the like may be suitably used.

In the present invention, the seeding density of the cells in the suspension culture is not particularly limited as long as the seeding density allows the cells to form aggregates, but the seeding density is preferably 1 × 10⁵ to 1 × 10⁷ cells/mL. The seeding density of the cells is preferably 2 × 10⁵ cells/mL or more, 3 × 10⁵ cells/mL or more, 4 × 10⁵ cells/mL or more, or 5 × 10⁵ cells/mL or more, and is preferably 9×10⁶ cells/mL or less, 8 × 10⁶ cells/mL or less, 7 × 10⁶ cells/mL or less, 6 × 10⁶ cells/mL or less, 5 × 10⁶ cells/mL or less, 4 × 10⁶ cells/mL or less, 3 × 10⁶ cells/mL or less, 2 × 10⁶ cells/mL or less, 1.9 × 10⁶ cells/mL or less, 1.8 × 10⁶ cells/mL or less, 1.7 × 10⁶ cells/mL or less, 1.6 × 10⁶ cells/mL or less, or 1.5 × 10⁶ cells/mL or less. In particular, a cell density in the range from 5 × 10⁵ cells/mL to 1.5 × 10⁶ cells/mL is preferable.

The cell aggregates include hundreds to thousands of cells per aggregate. In the present invention, the size (diameter) of the cell aggregates is not particularly limited and may, for example, be 50 µm or larger, 55 µm or larger, 60 µm or larger, 65 µm or larger, 70 µm or larger, 80 µm or larger, 90 µm or larger, 100 µm or larger, 110 µm or larger, 120 µm or larger, 130 µm or larger, 140 µm or larger, or 150 µm or larger, and may be 1000 µm or smaller, 900 µm or smaller, 800 µm or smaller, 700 µm or smaller, 600 µm or smaller, 500 µm or smaller, or 400 µm or smaller. Cell aggregates having a diameter of 150 µm to 400 µm are favorable for the present invention. Cell aggregates having diameters outside the above range may be mixed together.

The amount of the culture solution in the suspension culture can be appropriately adjusted in accordance with the culture vessel. For example, when a 12-well plate (the bottom surface area of each well in plan view being 3.5 cm²) is used, the amount may be 0.5 ml/well or more, and 1.5 ml/well or less, more preferably 1 ml/well. For example, when a 6-well plate (the bottom surface area of each well in plan view being 9.6 cm²) is used, the amount may be 1.5 mL/well or more, preferably 2 mL/well or more, and more preferably 3 mL/well or more, and may be 6.0 mL/well or less, preferably 5 mL/well or less, and more preferably 4 mL/well or less. For example, when a 125 mL Erlenmeyer flask (an Erlenmeyer flask having a capacity of 125 mL) is used, the amount may be 10 mL/vessel or more, is preferably 15 mL/vessel or more, more preferably 20 mL/vessel or more, more preferably 25 mL/vessel or more, and more preferably 30 mL/vessel or more, and may be 50 mL/vessel or less, is more preferably 45 mL/vessel or less, and more preferably 40 mL/vessel or less. For example, when a 500 mL Erlenmeyer flask (an Erlenmeyer flask having a capacity of 500 mL) is used, the amount may be 100 mL/vessel or more, preferably 105 mL/vessel or more, more preferably 110 mL/vessel or more, more preferably 115 mL/vessel or more, and more preferably 120 mL/vessel or more, and may be 150 mL/vessel or less, more preferably 145 mL/vessel or less, more preferably 140 mL/vessel or less, more preferably 135 mL/vessel or less, more preferably 130 mL/vessel or less, and more preferably 125 mL/vessel or less. For example, when a 1000 mL Erlenmeyer flask (an Erlenmeyer flask having a capacity of 1000 mL) is used, the amount may be 250 mL/vessel or more, preferably 260 mL/vessel or more, more preferably 270 mL/vessel or more, more preferably 280 mL/vessel or more, and more preferably 290 mL/vessel or more, and may be 350 mL/vessel or less, more preferably 340 mL/vessel or less, more preferably 330 mL/vessel or less, more preferably 320 mL/vessel or less, and more preferably 310 mL/vessel or less. For example, when a 2000 mL Erlenmeyer flask (an Erlenmeyer flask having a capacity of 2000 mL) is used, the amount may be 500 mL/vessel or more, more preferably 550 mL/vessel or more, and more preferably 600 mL/vessel or more, and may be 1000 mL/vessel or less, more preferably 900 mL/vessel or less, more preferably 800 mL/vessel or less, and more preferably 700 mL/vessel or less. For example, when a 3000 mL Erlenmeyer flask (an Erlenmeyer flask having a capacity of 3000 mL) is used, the amount may be 1000 mL/vessel or more, preferably 1100 mL/vessel or more, more preferably 1200 mL/vessel or more, more preferably 1300 mL/vessel or more, more preferably 1400 mL/vessel or more, and more preferably 1500 mL/vessel or more, and may be 2000 mL/vessel or less, more preferably 1900 mL/vessel or less, more preferably 1800 mL/vessel or less, more preferably 1700 mL/vessel or less, and more preferably 1600 mL/vessel or less. For example, when a 2 L culture bag (a disposable culture bag having a capacity of 2 L) is used, the amount may be 100 mL/bag or more, more preferably 200 mL/bag or more, more preferably 300 mL/bag or more, more preferably 400 mL/bag or more, more preferably 500 mL/bag or more, more preferably 600 mL/bag or more, more preferably 700 mL/bag or more, more preferably 800 mL/bag or more, more preferably 900 mL/bag or more, and more preferably 1000 mL/bag or more, and may be 2000 mL/bag or less, more preferably 1900 mL/bag or less, more preferably 1800 mL/bag or less, more preferably 1700 mL/bag or less, more preferably 1600 mL/bag or less, more preferably 1500 mL/bag or less, more preferably 1400 mL/bag or less, more preferably 1300 mL/bag or less, more preferably 1200 mL/bag or less, and more preferably 1100 mL/bag or less. For example, when a 10 L culture bag (a disposable culture bag having a capacity of 10 L) is used, the amount may be 500 mL/bag or more, more preferably 1 L/bag or more, more preferably 2 L/bag or more, more preferably 3 L/bag or more, more preferably 4 L/bag or more, and more preferably 5 L/bag or more, and may be 10 L/bag or less, more preferably 9 L/bag or less, more preferably 8 L/bag or less, more preferably 7 L/bag or less, and more preferably 6 L/bag or less. For example, when a 20 L culture bag (a disposable culture bag having a capacity of 20 L) is used, the amount may be 1 L/bag or more, more preferably 2 L/bag or more, more preferably 3 L/bag or more, more preferably 4 L/bag or more, more preferably 5 L/bag or more, more preferably 6 L/bag or more, more preferably 7 L/bag or more, more preferably 8 L/bag or more, more preferably 9 L/bag or more, and more preferably 10 L/bag or more, and may be 20 L/bag or less, more preferably 19 L/bag or less, more preferably 18 L/bag or less, more preferably 17 L/bag or less, more preferably 16 L/bag or less, more preferably 15 L/bag or less, more preferably 14 L/bag or less, more preferably 13 L/bag or less, more preferably 12 L/bag or less, and more preferably 11 L/bag or less. For example, when a 50 L culture bag (a disposable culture bag having a capacity of 50 L) is used, the amount may be 1 L/bag or more, more preferably 2 L/bag or more, more preferably 5 L/bag or more, more preferably 10 L/bag or more, more preferably 15 L/bag or more, more preferably 20 L/bag or more, and more preferably 25 L/bag or more, and may be 50 L/bag or less, more preferably 45 L/bag or less, more preferably 40 L/bag or less, more preferably 35 L/bag or less, and more preferably 30 L/bag or less. When the amount of the culture solution is within these ranges, cell aggregates of the appropriate size can be easily formed.

The capacity of the culture vessel that is used may be selected as appropriate and is not particularly limited, but in terms of the area, when seen in plan view, of the bottom surface of the portion in which the liquid culture medium is contained, the lower limit may, for example, be 0.32 cm², preferably 0.65 cm², more preferably 0.95 cm², even more preferably 1.9 cm², still more preferably 3.0 cm², 3.5 cm², 9.0 cm², or 9.6 cm², and the upper limit may, for example, be 1000 cm², preferably 500 cm², more preferably 300 cm², more preferably 150 cm², more preferably 75 cm², still more preferably 55 cm², even more preferably 25 cm², even more preferably 21 cm², and yet more preferably 9.6 cm², or 3.5 cm².

The culture temperature is not particularly limited as long as it is a culture temperature suitable for culturing the pluripotent stem cells that are used. Generally, the temperature is 30°C to 40°C, and is preferably about 37°C.

The cells are preferably cultured by using a CO₂ incubator or the like in an atmosphere with a CO₂ concentration of about 1% to 10%, preferably 5%.

### [7] Preculturing of pluripotent stem cells

Before inducing the differentiation of the above-mentioned pluripotent stem cell aggregates or pluripotent stem cells into endodermal cells, they may be suspension cultured by using a culture medium containing 2-mercaptoethanol and stauprimide to prepare a cell population.

The culture medium used in the preculture may, in accordance with the type of cells, be an MEM medium, a BME medium, a DMEM medium, a DMEM/F12 medium, an αMEM medium, an IMDM medium, an ES medium, a DM-160 medium, a Fisher medium, an F12 medium, a WE medium, an RPMI1640 medium, an Essential 6^{™} medium (Thermo Fisher Scientific), or the like.

The pluripotent stem cells are precultured in a suspension culture. The above-mentioned suspension culture conditions may be used, and furthermore, the cells may be suspension cultured by being adhered to a microcarrier or the like in advance or may be suspension cultured in the form of cell clumps composed only of cells, or a polymer such as collagen may be intermixed into the cell clumps. Thus, the form of the preculture is not particularly limited.

The concentration of 2-mercaptoethanol in the culture medium used for the preculture is not particularly limited as long as it is within a range in which the efficiency of differentiation induction increases. For example, the concentration of 2-mercaptoethanol is preferably 1 µM or more, 2 µM or more, 5 µM or more, 10 µM or more, 20 µM or more, 30 µM or more, 40 µM or more, or 50 µM or more, and preferably 200 µM or less, 150 µM or less, 120 µM or less, 100 µM or less, 90 µM or less, 80 µM or less, 70 µM or less, or 60 µM or less.

The concentration of stauprimide in the culture medium used for the preculture is not particularly limited as long as it is within a range in which the efficiency of differentiation induction increases. For example, the concentration of stauprimide is preferably 0.01 µmol/L, more preferably 0.02 µmoL/L, more preferably 0.05 µmoL/L, and more preferably 0.1 µmoL/L. The upper limit of the amount of stauprimide contained in the culture medium is preferably 10 µmol/L, more preferably 5 µmoL/L, more preferably 1 µmol/L, more preferably 0.8 µmol/L, more preferably 0.5 µmoL/L, more preferably 0.4 µmol/L, more preferably 0.3 µmoL/L, more preferably 0.2 µmοL/L, and more preferably 0.1 µmoL/L.

The culture medium used for the preculture should also preferably be a culture medium to which FGF2 (fibroblast growth factor 2) is not added. In some cases, the efficiency of differentiation to endodermal cells can be increased by using a culture medium to which FGF2 is not added.

The culture medium used for the preculture should also preferably be a culture medium to which TGF-β1 (transforming growth factor β1) is not added. In some cases, the efficiency of differentiation to endodermal cells can be increased by using a culture medium to which TGF-β1 is not added.

The culture medium used for the preculture should also preferably be a culture medium to which a WNT signaling activator is not added. In some cases, the efficiency of differentiation to endodermal cells can be increased by using a culture medium to which a WNT signaling activator is not added.

The culture medium used for the preculture should also preferably be a culture medium to which activin A (may be referred to alternatively as "ACTIVIN A" in the present description) is not added. In some cases, the efficiency of differentiation to endodermal cells can be increased by using a culture medium to which activin A is not added.

Amino acids, antibiotics, antioxidants, and other additives may also be added to the culture medium used for the preculture. For example, it is possible to add 0.1% to 2% (volume/volume) of NEAA (non-essential amino acids), 0.1% to 2% (volume/volume) of penicillin/streptomycin, 0.1 to 20 mg/mL of BSA or 1% to 25% (volume/volume) (preferably 1 % to 20% (volume/volume)) of KnockOut serum replacement (KSR), or the like.

The culture temperature is not particularly limited as long as it is a culture temperature suitable for culturing the pluripotent stem cells that are used. Generally, the temperature is 30°C to 40°C, and is preferably about 37°C.

The cells are preferably cultured by using a CO₂ incubator or the like in an atmosphere with a CO₂ concentration of about 1% to 10%, preferably 5%.

The culture period of the preculture of pluripotent stem cells is not particularly limited as long as it is a number of days allowing the cells to be cultured until the pluripotency is increased. For example, it is sufficient that the period not exceed 1 week. More specifically, the culture period may be shorter than 6 days, shorter than 5 days, shorter than 4 days, shorter than 3 days, or 6 hours to 48 hours, about 12 hours to 36 hours, or 18 hours to 24 hours.

### [8] Induced differentiation into endodermal cells

In the present invention, the cell population obtained by the above-described preculture is cultured under conditions that allow induced differentiation to endodermal cells, thereby inducing differentiation into endodermal cells.

Endodermal cells have the ability to differentiate into the tissues of organs such as the digestive tract, the lung, the thyroid gland, the pancreas, and the liver, the cells of secretory glands opening onto the digestive tract, and the peritoneum, the pleura, the larynx, the auditory tube, the trachea, the bronchi, and the urinary tract (most of the bladder and the urethra, and part of the ureter). In general, they are sometimes referred to as the definitive endoderm (DE). Differentiation from pluripotent stem cells to endodermal cells can be confirmed by measuring the expression levels of genes specific to endodermal cells. Examples of genes specific to endodermal cells include SOX17, FOXA2, CXCR4, AFP, GATA4, HOMES, and the like.

When inducing the pluripotent stem cells to differentiate into endodermal cells, the pluripotent stem cells are cultured by using a differentiation induction medium.

The differentiation induction medium is not particularly limited as long as it is a culture medium that induces the differentiation of pluripotent stem cells. Examples thereof include serum-containing media and serum-free media containing serum replacement components.

In accordance with the type of cells being used, it is possible to use a primate ES/iPS cell culture medium (ReproCELL medium), a BME medium, a BGJb medium, a CMRL 1066 medium, a Glasgow MEM medium, an Improved MEM Zinc Option medium, an IMDM medium, a Medium 199 medium, an Eagle MEM medium, an αMEM medium, a DMEM medium, a Ham's medium, an RPMI1640 medium, a Fischer's medium, and culture media obtained by mixing two or more media arbitrarily selected from these media. The culture medium is not particularly limited as long as it is a culture medium that can be used to culture animal cells.

The differentiation induction medium may contain a serum component or a serum replacement component. Examples of the serum component or the serum replacement component include albumin, insulin, transferrin, fatty acids, collagen precursors, trace elements (for example, zinc or selenium), B-27 Supplement (Thermo Fisher Scientific), N2 Supplement, N21 Supplement (R&D Systems), NeuroBrew-21 supplement (Miltenyi Biotec), KnockOut serum replacement (KSR), 2-mercaptoethanol, 3'thiolglycerol, and equivalents thereof.

Various additives, antibiotics, antioxidants, and the like may be further added to the differentiation induction medium. For example, it is possible to add 0.1 mM to 5 mM of sodium pyruvate, 0.1% to 2% (volume/volume) of non-essential amino acids, 0.1% to 2% (volume/volume) of penicillin, 0.1% to 2% (volume/volume) of streptomycin, and 0.1% to 2% (volume/volume) of amphotericin B, catalase, glutathione, galactose, retinoic acid (vitamin A), superoxide dismutase, ascorbic acid (vitamin C), D-α-tocopherol (vitamin E), and the like.

A differentiation-inducing factor is further added to the differentiation induction medium. Details regarding the differentiation-inducing factor will be described below.

The pluripotent stem cells are preferably cultured in a suspension culture during the induced differentiation. The cells may be suspension cultured by being adhered to a microcarrier or the like or may be suspension cultured in the form of cell clumps composed only of cells, or a polymer such as collagen may be intermixed into the cell clumps. Thus, the form of the culture is not particularly limited.

The culture temperature used when culturing the cells to induce differentiation is not particularly limited as long as it is a culture temperature suitable for culturing the pluripotent stem cells that are used. Generally, the temperature is 30°C to 40°C, and is preferably about 37°C.

The cells are preferably cultured by using a CO₂ incubator or the like in an atmosphere with a CO₂ concentration of about 1% to 10%, preferably 5%.

The culture period for the differentiation culture from the pluripotent stem cells to endodermal cells is not particularly limited as long as the cells are converted to a cell type in which the cell properties of endodermal cells are exhibited. For example, it is sufficient for the period to be within 2 weeks. More specifically, the culture period may be 2 days or longer and 8 days or shorter, more preferably 2 days or longer and 7 days or shorter, and even more preferably 3 days or longer and 6 days or shorter. As an example, the culture period may be 4 or 5 days.

### [9] Differentiation-inducing factor used to induce differentiation into endodermal cells, and other additives

Preferably, the endodermal cells are endodermal cells that have been induced to differentiate by culturing pluripotent stem cells in a culture medium containing a TGFβ (transforming growth factor β) superfamily signaling activator, and thereafter culturing the cells in a culture medium to which FGF2 and BMP4 (bone morphogenetic protein 4) are not added.

When activin A is used in the culture medium containing a TGFβ superfamily signaling activator, the initial concentration of activin A added is preferably 1 ng/mL or more, 2 ng/mL or more, 3 ng/mL or more, 5 ng/mL or more, 10 ng/mL or more, 20 ng/mL or more, 30 ng/mL or more, 40 ng/mL or more, or 50 ng/mL or more, and preferably 1,000 ng/mL or less, 900 ng/mL or less, 800 ng/mL or less, 700 ng/mL or less, 600 ng/mL or less, 500 ng/mL or less, 400 ng/mL or less, 300 ng/mL or less, 200 ng/mL or less, 150 ng/mL or less, or 100 ng/mL or less.

When FGF2 is used in the culture medium containing a TGFβ superfamily signaling activator, the initial concentration of FGF2 added is preferably 1 ng/mL or more, 2 ng/mL or more, 3 ng/mL or more, 5 ng/mL or more, 10 ng/mL or more, 20 ng/mL or more, 30 ng/mL or more, or 40 ng/mL or more, and preferably 1,000 ng/mL or less, 900 ng/mL or less, 800 ng/mL or less, 700 ng/mL or less, 600 ng/mL or less, 500 ng/mL or less, 400 ng/mL or less, 300 ng/mL or less, 200 ng/mL or less, 150 ng/mL, 100 ng/mL or less, 90 ng/mL or less, 80 ng/mL or less, or 70 ng/mL or less.

When BMP4 is used in the culture medium containing a TGFβ superfamily signaling activator, the initial concentration of BMP4 added is preferably 1 ng/mL or more, 2 ng/mL or more, 3 ng/mL or more, 5 ng/mL or more, 6 ng/mL or more, 7 ng/mL or more, 8 ng/mL or more, 9 ng/mL or more, 10 ng/mL or more, 11 ng/mL or more, 12 ng/mL or more, 13 ng/mL or more, 14 ng/mL or more, or 15 ng/mL or more, and preferably 1,000 ng/mL or less, 900 ng/mL or less, 800 ng/mL or less, 700 ng/mL or less, 600 ng/mL or less, 500 ng/mL or less, 400 ng/mL or less, 300 ng/mL or less, 200 ng/mL or less, 150 ng/mL, 100 ng/mL or less, 90 ng/mL or less, 80 ng/mL or less, 70 ng/mL or less, 60 ng/mL or less, 50 ng/mL or less, 40 ng/mL or less, or 30 ng/mL or less.

The culture medium to which FGF2 and BMP4 are not added preferably contains activin A.

When the culture medium to which FGF2 and BMP4 are not added contains activin A, the initial concentration of activin A added is preferably 1 ng/mL or more, 2 ng/mL or more, 3 ng/mL or more, 5 ng/mL or more, 10 ng/mL or more, 20 ng/mL or more, 30 ng/mL or more, 40 ng/mL or more, or 50 ng/mL or more, and preferably 1,000 ng/mL or less, 900 ng/mL or less, 800 ng/mL or less, 700 ng/mL or less, 600 ng/mL or less, 500 ng/mL or less, 400 ng/mL or less, 300 ng/mL or less, 200 ng/mL or less, 150 ng/mL or less, or 100 ng/mL or less.

The culture medium to which FGF2 and BMP4 are not added preferably contains at least one or more substances selected from the group consisting of insulin, transferrin, sodium selenite, and ethanolamine.

The concentration of insulin added is preferably 0.001 µg/mL or more, 0.01 µg/mL or more, 0.05 µg/mL or more, 0.1 µg/mL or more, or 0.2 µg/mL or more, and preferably 10,000 µg/mL or less, 1,000 µg/mL or less, 100 µg/mL or less, 10 µg/mL or less, 9 µg/mL or less, 8 µg/mL or less, 7 µg/mL or less, 6 µg/mL or less, 5 µg/mL or less, 4 µg/mL or less, 3 µg/mL or less, or 2 µg/mL or less. The concentration of transferrin added is preferably 0.001 µg/mL or more, 0.01 µg/mL or more, 0.05 µg/mL or more, 0.06 µg/mL or more, 0.07 µg/mL or more, 0.08 µg/mL or more, 0.09 µg/mL or more, 0.1 µg/mL or more, or 0.11 µg/mL or more, and preferably 10,000 µg/mL or less, 1,000 µg/mL or less, 100 µg/mL or less, 10 µg/mL or less, 9 µg/mL or less, 8 µg/mL or less, 7 µg/mL or less, 6 µg/mL or less, 5 µg/mL or less, 4 µg/mL or less, 3 µg/mL or less, 2 µg/mL or less, 1.9 µg/mL or less, 1.8 µg/mL or less, 1.7 µg/mL or less, 1.6 µg/mL or less, 1.5 µg/mL or less, 1.4 µg/mL or less, 1.3 µg/mL or less, 1.2 µg/mL or less, or 1.1 µg/mL or less. The concentration of sodium selenite added is preferably 0.001 ng/mL or more, 0.01 ng/mL or more, or 0.1 ng/mL or more, and preferably 10,000 ng/mL or less, 1,000 ng/mL or less, 100 ng/mL or less, 10 ng/mL or less, or 1 ng/mL or less. The concentration of ethanolamine added is preferably 0.001 µg/mL or more, 0.01 µg/mL or more, 0.02 µg/mL or more, 0.03 µg/mL or more, or 0.04 µg/mL or more, and preferably 10,000 µg/mL or less, 1,000 µg/mL or less, 100 µg/mL or less, 10 µg/mL or less, 1 µg/mL or less, 0.9 µg/mL or less, 0.8 µg/mL or less, 0.7 µg/mL or less, 0.6 µg/mL or less, 0.5 µg/mL or less, or 0.4 µg/mL or less.

It is preferable for the culture medium containing a TGFβ superfamily signaling activator and/or the culture medium to which FGF2 and BMP4 are not added to further contain 2-mercaptoethanol. The action of 2-mercaptoethanol can raise the efficiency of induced differentiation to endodermal cells.

It is preferable for the culture medium containing a TGFP superfamily signaling activator to further contain a WNT signaling activator.

When CHIR99021 is used in the culture medium containing a TGFP superfamily signaling activator, the initial concentration added is preferably 0.01 µM or more, 0.02 µM or more, 0.03 µM or more, 0.04 µM or more, 0.05 µM or more, 0.1 µM or more, 0.2 µM or more, 0.3 µM or more, 0.4 µM or more, 0.5 µM or more, 0.6 µM or more, 0.7 µM or more, 0.8 µM or more, 0.9 µM or more, 1 µM or more, or 2 µM or more, and preferably 100 µM or less, 90 µM or less, 80 µM or less, 70 µM or less, 60 µM or less, 50 µM or less, 45 µM or less, 40 µM or less, 35 µM or less, 30 µM or less, 25 µM or less, 20 µM or less, 15 µM or less, 10 µM or less, or 5 µM or less. More preferably, the initial concentration is 3 µM or 4 µM.

The culture medium containing a TGFβ superfamily signaling activator and/or the culture medium to which FGF2 and BMP4 are not added contains at least glucose. The lower limit of the concentration of glucose contained in the culture medium is not particularly limited as long as it is a concentration at which the cells can proliferate, but it should preferably be 0.01 g/L or more. Additionally, the upper limit of the concentration of glucose contained in the culture medium is not particularly limited as long as it is a concentration at which the cells do not die, but it should preferably be, for example, 10 g/L or less. As another embodiment, a culture medium containing less than 2.0 g/L of glucose is preferable for the purposes of achieving efficient differentiation to endodermal somatic cells. The glucose concentration in the culture medium containing a TGFβ superfamily signaling activator and/or the culture medium to which FGF2 and BMP4 are not added may be 1.0 g/L or less, 0.9 g/L or less, 0.8 g/L or less, 0.7 g/L or less, or 0.6 g/L or less. The lower limit of the glucose concentration in the case in which the culture medium containing a TGFβ superfamily signaling activator and/or the culture medium to which FGF2 and BMP4 are not added contains glucose is not particularly limited, and may be 0.01 g/L or more, 0.02 g/L or more, 0.05 g/L or more, 0.1 g/L or more, 0.2 g/L or more, 0.3 g/L or more, 0.4 g/L or more, or 0.5 g/L or more.

### [10] Use of pancreatic α cells

The pancreatic α cells obtained by the method of the present invention have a glucagon-secretory ability, and are useful for elucidating the glucagon-secretory mechanism in pancreatic islets and as a development tool of a novel therapeutic agent for diabetes. That is, the pancreatic α cells produced by using the method of the present invention can be used for screening of substances that inhibit the secretion of glucagon from the cells. The substances obtained as a result of this screening, which are substances that inhibit glucagon secretion from the pancreatic α cells, can be used as a novel therapeutic agent for diabetes.

Hereinafter, the present invention will be explained in detail by providing examples, but the present invention is not limited to these examples.

### EXAMPLES

### [Comparative Example 1]

Human iPS cells were induced to differentiate into pancreatic β cells by referring to S. G. Yabe et al., Induction of functional islet-like cells from human iPS cells by suspension culture, Regen. Ther., 10, 69-76, 2019.

### (1) Maintenance culture of human iPS cells

Human iPS cells were subjected to an undifferentiated-state maintenance culture using a DMEM/HAM'S F12 medium containing 20 (volume/volume)% KnockOut serum replacement (KSR), 1 × non-essential amino acids (NEAA), 55 µM 2-mercaptoethanol (2-ME), 7.5 ng/mL recombinant human fibroblast growth factor (FGF2), and 0.5 × penicillin and streptomycin (PS) on SNL feeder cells treated with mitomycin-C. Alternatively, an undifferentiated-state maintenance culture was performed on a plate coated with Vitronectin in a StemFit medium containing 0.5 × penicillin and streptomycin. The cells were cultured by adding Y-27632 in such a way that the final concentration was 10 µM only at the time of seeding. The culture was implemented in a 5% CO₂ incubator at 37°C.

### (2) Production of human iPS cell clumps

Human iPS cells were washed once with PBS (-), and then Accumax was added. After incubating at 37°C for 5 to 15 minutes, the cells were dispersed and recovered to a single cell by pipetting. The cells, numbering 4 × 10⁷, were suspended in 30 mL of an mTeSR1 medium containing 10 µM of Y-27632, and seeded in a 30 mL single-use bioreactor (ABLE Corporation). This was mounted on a six-channel magnetic stirrer (ABLE Corporation), and while stirring at the speed of 45 rpm, the suspension culture was implemented in a 5% CO₂ incubator at 37°C for 2 days.

### (3) Preculture of human iPS cells

The cell population that formed the clumps obtained in the production of the clumps was suspended in a DMEM/HAM'S F12 containing 20 (volume/volume)% KSR, 1 × NEAA, 0.1 µM stauprimide, and 55 µM 2-ME, and transferred to a 30 mL single-use bioreactor. This was mounted on a six-channel magnetic stirrer, and while stirring at the speed of 45 rpm, the suspension culture was implemented in a 5% CO₂ incubator at 37°C for 1 day.

### (4) Induced differentiation into pancreatic β cells

The cell population obtained by the preculture was first induced to differentiate into endodermal cells. Specifically, for the first 2 days, the cells were suspension cultured in an RPMI1640 medium containing 0.25 (volume/volume)% bovine serum albumin (BSA), 1 mM sodium pyruvate, 1 × NEAA, 0.4 × PS, 80 ng/mL recombinant human activin A, 50 ng/mL FGF2, 20 ng/mL recombinant human bone morphogenetic protein 4 (BMP4), 3 µM CHIR99021, and 55 µM 2-ME. On the 3rd day, the suspension culture was implemented in a medium from which FGF2, BMP4, and CHIR99021 were removed, and on the 4th day, the suspension culture was implemented in a medium obtained by adding 0.5 (volume/volume)% KSR to the medium of the 3rd day, thereby inducing differentiation into endodermal cell. A 30 mL single-use bioreactor (ABLE Corporation) was mounted on a six-channel magnetic stirrer, and while stirring at the speed of 45 rpm, the suspension culture was implemented in a 5% CO₂ incubator at 37°C.

The endodermal cells obtained above were induced to differentiate into primitive gut tube (PGT) cells. Specifically, the suspension culture was implemented for 3 days in an RPMI1640 medium or a mixed medium containing a DMEM medium and a HAM'S F12 medium in 1: 1, containing 0.25 (volume/volume)% BSA, 1 mM sodium pyruvate, 1 × NEAA, 0.4 × PS, 1 (volume/volume)% B-27 supplement, 0.3 (volume/volume)% insurin-transferrin-selenium-ethanalumine (ITS-X), and 50 ng/mL recombinant human fibroblast growth factor 7 (FGF-7). A 30 mL single-use bioreactor was mounted on a six-channel magnetic stirrer, and while stirring at the speed of 45 rpm, the suspension culture was implemented in a 5% CO₂ incubator at 37°C.

The primitive gut tube (PGT) cells obtained above were induced to differentiate into posterior foregut (PFG) cells. Specifically, the suspension culture was implemented for 4 days in an RPM11640 medium or a mixed medium containing a DMEM medium and a HAM'S F12 medium in 3:1, containing 0.15 (volume/volume)% BSA, 1 × NEAA, 0.4 × PS, 1 (volume/volume)% B-27 supplement, 0.3 (volume/volume)% ITS-X, 50 ng/mL FGF-7, 0.3 µM indolactam V (ILV), 0.2 µM LDN193189, 0.25 µM SANT1, and 0.25 mM ascorbic acid. A 30 mL single-use bioreactor was mounted on a six-channel magnetic stirrer, and while stirring at the speed of 45 rpm, the suspension culture was implemented in a 5% CO₂ incubator at 37°C.

Subsequently, the posterior foregut (PGT) cells obtained above were induced to differentiate into pancreatic progenitor (PP) cells. Specifically, the suspension culture was implemented for 3 days in a DMEM medium or a mixed medium containing a DMEM medium and a HAM'S F12 medium in 4:1, containing 0.15 (volume/volume)% BSA, 1 × NEAA, 0.4 × PS, 1 (volume/volume)% B-27 supplement, 0.25 (volume/volume)% ITS-X, 50 ng/mL recombinant human fibroblast growth factor 10 (FGF10), 0.04 µM EC23, 0.2 µM LDN193189, 0.25 µM SANT1, 5 µM zinc sulfate, 55 µM 2-ME, and 0.25 mM ascorbic acid. A 30 mL single-use bioreactor was mounted on a six-channel magnetic stirrer, and while stirring at the speed of 45 rpm, the suspension culture was implemented in a 5% CO₂ incubator at 37°C.

Subsequently, the pancreatic progenitor (PP) cells were induced to differentiate into pancreatic endocrine precursor (EP) cells. Specifically, the suspension culture was implemented for 7 days in a DMEM medium or a mixed medium containing a DMEM medium and a HAM'S F12 medium in 4:1, containing 0.15 (volume/volume)% BSA, 1 × NEAA, 0.4 ×PS, 1 (volume/volume)% B-27 supplement, 0.3 (volume/volume)% ITS-X, 20 ng/mL, EGF, 50 ng/mL exendin 4, 0.01 µM EC23, 0.2 µM LDN193189, 0.25 µM SANT1, 10 µM RepSox, 0.5 µM DBZ, 10 µM Y-27632, 5 µM zinc sulfate, 10 µg/mL heparin, and 5 mM nicotinamide. A 30 mL single-use bioreactor was mounted on a six-channel magnetic stirrer, and while stirring at the speed of 45 rpm, the suspension culture was implemented in a 5% CO₂ incubator at 37°C.

Finally, the pancreatic endocrine precursor (EP) cells were induced to differentiate into pancreatic β cells. Specifically, the suspension culture was implemented for 10 to 12 days in a DMEM medium or a mixed medium containing a DMEM medium and a HAM'S F12 medium in 4:1, containing 0.15 (volume/volume)% BSA, 1 × NEAA, 0.4 × PS, 1 (volume/volume)% B-27 supplement, 0.3 (volume/volume)% ITS-X, 50 ng/mL exendin, 50 ng/mL human recombinant insulin-like growth factor (IGF), 5 µM forskolin, 5 µM zinc sulfate, 10 µM RepSox, 10 µg/mL heparin, and 5 mM nicotinamide. A 30 mL single-use bioreactor was mounted on a six-channel magnetic stirrer, and while stirring at the speed of 45 rpm, the suspension culture was implemented in a 5% CO₂ incubator at 37°C. Alternatively, a 100 mL single-use bioreactor (ABLE Corporation) was mounted on a microbial culture apparatus (BMZ-P, ABLE Corporation), and while stirring at the speed of 50 rpm, the suspension culture was implemented in a 5% CO₂ environment at 37°C.

### [Example 1]

### (1) Induction of differentiation

Differentiation was induced by the same method as in Comparative Example 1 up to the endodermal cells. For induction of differentiation from the endodermal cells into primitive gut tube (PGT) cells, the suspension culture was implemented for 3 days in a medium obtained by adding 0.67 µM LDN193189 to the medium used in Comparative Example 1, a medium obtained by adding 1 µM EC23 to the medium used in Comparative Example 1, or a medium obtained by adding both of them to the medium used in Comparative Example 1. In addition, 2.5 µM Y-27632 was added to the above-mentioned medium for the purpose of maintaining the shape of the clumps. A 30 mL single-use bioreactor was mounted on a six-channel magnetic stirrer, and while stirring at the speed of 45 rpm, the suspension culture was implemented in a 5% CO₂ incubator at 37°C. Induction of differentiation after posterior foregut (PFG) cells was performed in the same method as in Comparative Example 1.

### (2) <Quantitative RT-PCR>

Total RNA was isolated and purified from the cells obtained by inducing differentiation in (1) using Isogen (Nippon Gene), and cDNA was synthesized from the obtained total RNA using PrimeScriptII (TaKaRa Bio Inc.). Using the obtained cDNA as a template, quantitative PCR was implemented by means of a MyiQ qPCR machine (Bio-Rad), using GoTaq qPCR master mix (Promega). For the detection, the intercalation method using SYBR Green was used, and for the gene expression level comparison, the relative quantification method by means of the ΔΔCt method or the ΔCt method was used. The expression level of each gene was standardized by OAZ1 which is a housekeeping gene.

The base sequences of the primers used in the quantitative PCR are as indicated below.
OAZ1 F: GTC AGA GGG ATC ACAATC TTT CAG (SEQ ID NO: 1)
OAZ1 R: GTC TTG TCG TTG GAC GTT AGT TC (SEQ ID NO: 2)
INS F: TTG TGA ACC AAC ACC TGT GC (SEQ ID NO: 3)
INS R: GTG TGT AGA AGC CTC GTT CC (SEQ ID NO: 4)
GCG F: ACA TTC ACC AGT GAC TAC AGC AAG (SEQ ID NO: 5)
GCG R: GGC AAT GTT ATT CCT GTT CCT C (SEQ ID NO: 6)
NKX6.1 F: ATC TTC GCC CTG GAG AAG AC (SEQ ID NO: 7)
NKX6.1 R: CGT GCT TCT TCC ACT TG (SEQ ID NO: 8)

### <Measurement results>

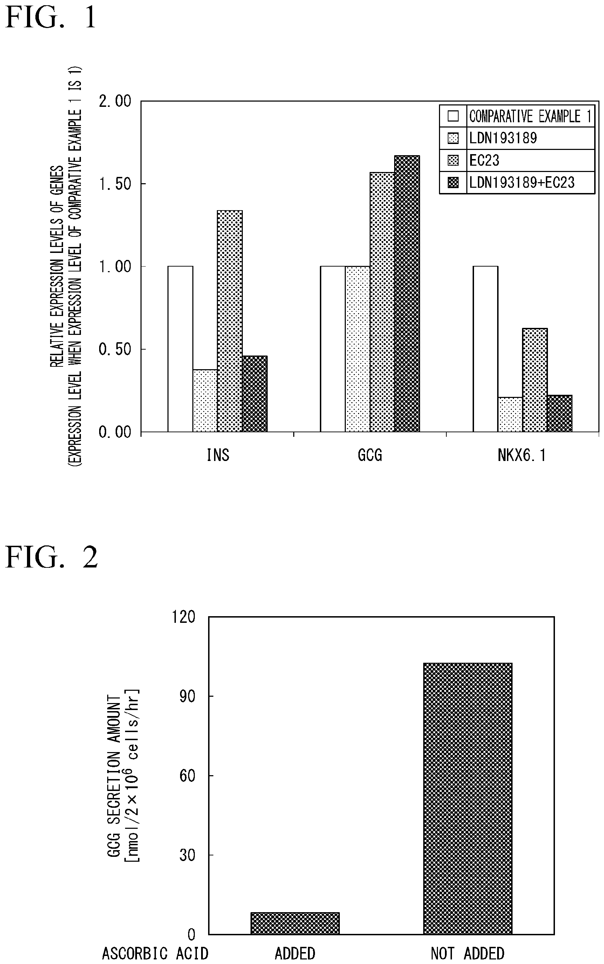

The measurement results of the gene expression levels are shown in Fig. 1.

When the cells obtained under each condition of Example 1 were compared to the cells obtained by the method of Comparative Example 1, a down-regulation was recognized in the expression levels of the INS gene and the NKX6.1 gene which are genes specific to pancreatic β cells under the condition in which LDN193189 was added. On the other hand, an up-regulation was recognized in the expression level of the GCG gene which is a gene specific to pancreatic α cells under the condition in which EC23 was added. Based on this, it became clear that, when inducing differentiation from endodermal cells into primitive gut tube (PGT) cells, addition of LDN193189 inhibits the induction of differentiation into pancreatic β cells, and addition of EC23 promotes the induction of differentiation into pancreatic α cells.

Furthermore, under the condition in which both LDN193189 and EC23 were added, the expression levels of the INS gene and the NKX6.1 gene were down-regulated, and the expression levels of the GCG gene was up-regulated, which shows that pancreatic α cells were produced instead of pancreatic β cells (Fig. 1). Accordingly, the cells obtained in the present example are mainly pancreatic α cells, and pancreatic α cells can be efficiently produced by inducing differentiation from endodermal cells into primitive gut tube (PGT) cells by the method of Example 1.

### [Example 2]

### (1) Induction of differentiation

Primitive gut tube (PGT) cells were produced from human iPS cells by the same method as in Example 1. The obtained primitive gut tube (PGT) cells were induced to differentiate into cells after posterior foregut (PFG) cells using a medium obtained by removing ascorbic acid from the medium of Comparative Example 1.

### (2) Measurement of GCG secretion amount

The cells obtained by inducing differentiation in (1) were recovered in 2 × 10⁶ cells and cultured for 30 minutes in a DMEM medium (containing 2 mM glucose) containing 10 mM HEPES and 0.1 (volume/volume)% BSA. Thereafter, the cells were washed twice with a DMEM medium (without glucose) containing 10 mM HEPES and 0.1 (volume/volume)% BSA. Thereafter, the cells were cultured for 1 hour in a DMEM medium containing 1 mL of 2.0 mM glucose, 10 mM HEPES, and 0.1 (volume/volume)% BSA. Thereafter, the GCG concentration contained in the culture supernatant was measured using a Glucagon ELISA 10 µl Kit (Mercodia).

The GCG secretion amounts of the cells obtained in Example 1 and Comparative Example 1 were also measured as comparison targets by the above-mentioned method.

### (3) Quantitative RT-PCR

The cells obtained by inducing differentiation in (1) were subjected to quantitative PCR using the same method as the method described in Example 1. In the present example, the following primers were added to the primers of Example 1 and used for quantitative PCR.
ARX F: AAG GAG GTG TGC TAAAGG CTG (SEQ ID NO: 9)
ARX R: GCT GGT CCT CTG TTT CCA TTT G (SEQ ID NO: 10)
GC F: CTG AGT GCT GCA CCA AAG AG (SEQ ID NO: 11)
GC R: ATT TGT GGG TTC CAC GTA GGT AG (SEQ ID NO: 12)

### <Measurement results>

A significant difference was recognized in the glucagon secretion amounts between the cells obtained by the method of Example 1 and the cells obtained by the method of Example 2. Specifically, the glucagon secretion amount of the cells obtained by the method of Example 2 was larger than that of the cells obtained by the method of Example 1. That is, it can be seen that inducing differentiation from primitive gut tube cells into pancreatic α cells becomes efficient by removing ascorbic acid from the differentiation induction medium (or culturing in the differentiation induction medium not containing ascorbic acid) in the differentiation induction step after the primitive gut tube (PGT) cells (Fig. 2).

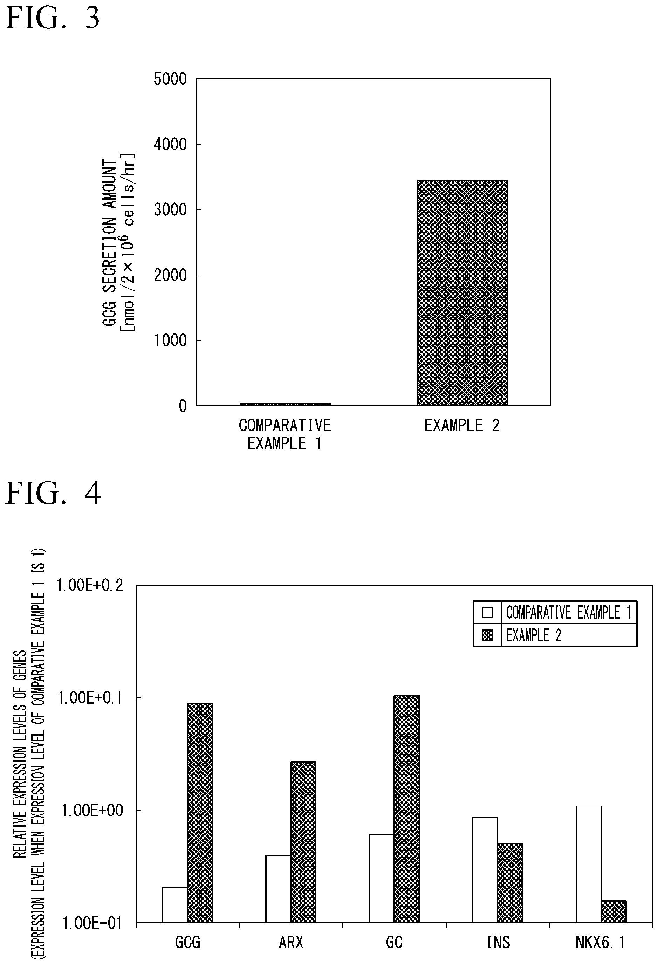

Similarly, when the cells obtained by the method of Example 2 were compared to the cells obtained by the method of Comparative Example 1 (Fig. 3), it can be seen that the glucagon secretion amount was significantly larger in the cells obtained by the method of Example 2 as compared to the cells obtained by the method of Comparative Example 1. Based on this, in addition to producing primitive gut tube (PGT) cells by the method of Example 1, by performing the subsequent differentiation induction step in the absence of ascorbic acid, pancreatic α cells can be more efficiently produced from human iPS cells.

Accordingly, when human iPS cells are induced to differentiate by the method described in the present invention, it is possible to suppress the differentiation into pancreatic β cells and promote the differentiation into pancreatic α cells, which makes it possible to efficiently produce pancreatic α cells.

Next, Fig. 4 shows the results of measuring the gene expression levels of the cells obtained by the method of Example 2 and the cells obtained by the method of Comparative Example 1. In the cells obtained by the method of Example 2, the expression levels of the GCG gene, the ARX gene, and the GC gene, which are genes specific to pancreatic α cells, were high, whereas the expression levels of the INS gene and the NKX6.1 gene, which are genes specific to pancreatic β cells, were low. Based on this, it can be seen that pancreatic α cells can be efficiently produced by inducing differentiation of pluripotent stem cells by the method of the present invention.

### [Example 3]

In Example 3, the influence of the oxygen concentration in the culture solution in the step of inducing differentiation from pancreatic endocrine precursor (EP) cells into pancreatic α cells was investigated.

### (1) Induction of differentiation

Cells were produced by the same method as in Comparative Example 1 up to pancreatic endocrine precursor (EP) cells. In the induction of differentiation from pancreatic endocrine precursor (EP) cells into pancreatic α cells, the medium of Comparative Example 1 was used, and the suspension culture was implemented for 12 days by using a microbial culture apparatus (BMZ-P, ABLE Corporation) while controlling the oxygen concentration in the culture solution. The dissolved oxygen concentration was controlled so that the dissolved oxygen concentration in the culture solution became the oxygen concentration shown in Table 1 when a saturated oxygen concentration in the culture solution at 1 atm was set to 100%.

**[Table 1]**

| Condition name | 0 to 2 days | 2 to 4 days | 4 to 6 days | 6 to 8 days | 8 to 10 days | 10 to 12 days |
|---|---|---|---|---|---|---|
| #3-1 | Non-controlled | 20% | 40% | 60% | 80% | 100% |
| #3-2 | Non-controlled | 20% | 40% | 60% | 60% | 60% |
| #3-3 | Non-controlled | 20% | 30% | 30% | 30% | 30% |

### (2) Measurement of oxygen concentration

The oxygen concentration in the culture solution was measured over time using a fluorescence type dissolved oxygen meter. The measured value indicates the amount of dissolved oxygen (%) when the saturated dissolved oxygen concentration in the culture solution at 1 atm was set to 100%.

### (3) <Quantitative RT-PCR>

In the stage of inducing the differentiation from pancreatic endocrine precursor (EP) cells into pancreatic α cells, the obtained cells were recovered every two days to perform quantitative RT-PCR by the method described in Example 1.

### <Measurement results>

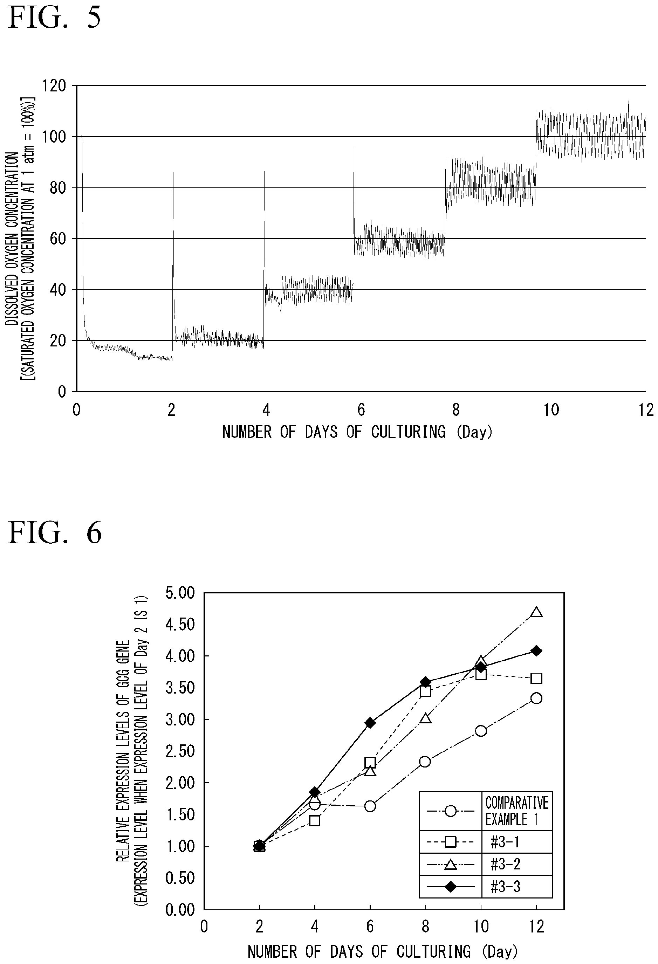

The oxygen concentration in the culture in #3-1 is shown in Fig. 5. When the oxygen concentration was not controlled, the amount of dissolved oxygen was about 15% due to oxygen consumption by cells. On the other hand, when the oxygen concentration was controlled using the culture apparatus, the dissolved oxygen concentration could be controlled at around ± 10% of the target oxygen concentration.

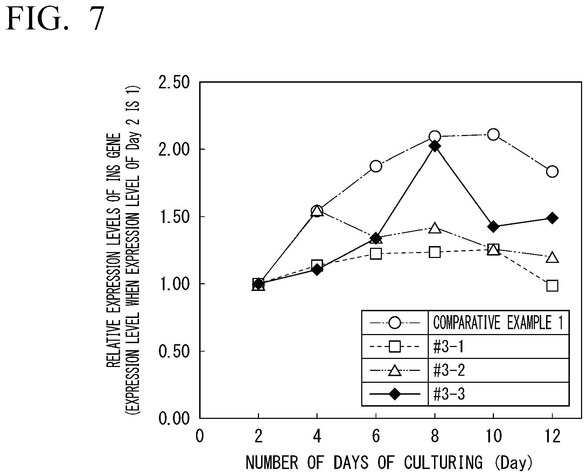

The results of measuring the gene expression level for the obtained cells are shown in Figs. 6 and 7. As compared to the oxygen concentration non-controlled group (Comparative Example 1), the expression level of the GCG gene, which is a gene specific to pancreatic α cells, was higher in the oxygen concentration controlled group (Example 3) (Fig. 6), and the expression level of the INS gene, which is a gene specific to pancreatic β cells, was lower in the oxygen concentration controlled group (Fig. 7).

Based on this, it was found that pancreatic α cells can be more efficiently produced if the oxygen concentration is appropriately controlled in the step of inducing the differentiation from pancreatic endocrine precursor (EP) cells into pancreatic α cells.

## Claims

1. A method for inducing differentiation into pancreatic α cells, the method comprising:
a step (a) of culturing endodermal cells, which have been induced to differentiate from pluripotent stem cells, in the presence of a bone morphogenetic protein (BMP) signaling inhibitor, and retinoic acid or a retinoic acid analog to induce differentiation into primitive gut tube (PGT) cells;
a step (b) of culturing the primitive gut tube (PGT) cells to induce differentiation into pancreatic endocrine precursor (EP) cells; and
a step (c) of culturing the pancreatic endocrine precursor (EP) cells to induce differentiation into pancreatic α cells,
wherein the step (b) and the step (c) are performed in the absence of ascorbic acid.

2. The method for inducing differentiation into pancreatic α cells according to Claim 1,
wherein the step (c) is performed under an oxygen supply condition.

3. The method for inducing differentiation into pancreatic α cells according to Claim 2,
wherein the oxygen supply condition is a condition in which a dissolved oxygen concentration in a culture solution is controlled to be 20% to 50% when a saturated dissolved oxygen concentration in the culture solution at 37°C at 1 atm is 100%.

4. The method for inducing differentiation into pancreatic α cells according to any one of Claims 1 to 3,
wherein the step (a) is performed in the presence of a ROCK signaling inhibitor.

5. The method for inducing differentiation into pancreatic α cells according to any one of Claims 1 to 4,
wherein the endodermal cells induced to differentiate from the pluripotent stem cells are endodermal cells induced to differentiate by culturing the pluripotent stem cells in a culture medium to which FGF2 and BMP4 are not added after culturing in a culture medium containing a TGFβ superfamily signaling activator.

6. The method for inducing differentiation into pancreatic α cells according to any one of Claims 1 to 5,
wherein the step (b) comprises
a step (b1) of culturing the primitive gut tube (PGT) cells in the presence of a protein kinase C (PKC) activator to induce differentiation into posterior foregut (PFG) cells,
a step (b2) of culturing the posterior foregut (PFG) cells in the presence of retinoic acid or an analog thereof to induce differentiation into pancreatic progenitor (PP) cells, and
a step (b3) of culturing the pancreatic progenitor (PP) cells in the presence of a Notch signaling inhibitor and a ROCK signaling inhibitor to induce differentiation into pancreatic endocrine precursor (EP) cells.

7. The method for inducing differentiation into pancreatic α cells according to any one of Claims 1 to 6,
wherein the step (c) comprises a step of culturing the pancreatic endocrine precursor (EP) cells in the presence of an insulin receptor signaling activator, transferrin, and selenous acid to induce differentiation into pancreatic α cells.

8. The method for inducing differentiation into pancreatic α cells according to any one of Claims 1 to 7,
wherein the culturing is performed by suspension culture.

9. The method for inducing differentiation into pancreatic α cells according to any one of Claims 2 to 8,
wherein the oxygen supply is performed under stirring culture.
